# EUROPEAN PATENT SPECIFICATION

(11) **EP 1 196 566 B1**
(45) Date of publication and mention of the grant of the patent: **01.02.2006**
(21) Application number: 00945309.3
(22) Date of filing: 11.07.2000
(51) Int. Cl.: C12N 15/12, C12N 15/10, C12N 15/62, C12N 15/65, C12N 15/69, C12N 15/85, C07K 14/435, C07K 14/71, C07K 16/42, C12Q 1/68

(54) **EXPRESSION VECTORS AND METHODS**
EXPRESSIONSVEKTOREN UND ANWENDUNGSMETHODEN
VECTEURS D'EXPRESSION ET PROCEDES

(30) Priority: 12.07.1999 US 143360 P
(43) Date of publication of application: 17.04.2002
(73) Proprietor: GENENTECH, INC., South San Francisco, CA 94080-4990 (US)
(72) Inventor: CHISHOLM, Vanessa, San Mateo, CA 94403 (US); CROWLEY, Craig, W., Portola Valley, CA 94028 (US); KRUMMEN, Lynne, A., San Francisco, CA 94116 (US); MENG, Yu-Ju, G., Albany, CA 94706 (US)
(74) Representative: Walton, Seán Malcolm
(86) International application number: PCT/US2000/018841
(87) International publication number: WO 2001/004306

(56) References cited:
- EP-A- 0 711 835
- WO-A-96/04391
- MENG Y GLORIA ET AL: "Green fluorescent protein as a second selectable marker for selection of high producing clones from transfected CHO cells." GENE (AMSTERDAM)., vol. 242, no. 1-2, 25 January 2000 (2000-01-25), pages 201-207, XP002151701 ISSN: 0378-1119
- LUCAS BRIAN K ET AL: "High-level production of recombinant proteins in CHO cells using a dicistronic DHFR intron expression vector." NUCLEIC ACIDS RESEARCH, vol. 24, no. 9, 1996, pages 1774-1779, XP002151702 ISSN: 0305-1048 cited in the application
- BENNETT ROBERT P ET AL: "Fusion of green fluorescent protein with the zeocin-resistance marker allows visual screening and drug selection of transfected eukaryotic cells." BIOTECHNIQUES, vol. 24, no. 3, March 1998 (1998-03), pages 478-482, XP002151703 ISSN: 0736-6205 cited in the application
- PRIMIG MICHAEL ET AL: "A novel GFPneo vector designed for the isolation and analysis of enhancer elements in transfected mammalian cells." GENE (AMSTERDAM), vol. 215, no. 1, 17 July 1998 (1998-07-17), pages 181-189, XP002151704 ISSN: 0378-1119 cited in the application
- MOSSER D D ET AL: "Use of a dicistronic expression cassette encoding the green fluorescent protein for the screening and selection of cells expressing inducible gene products." BIOTECHNIQUES, vol. 22, no. 1, 1997, pages 150-152, 154, 156, 158-161, XP002151705 ISSN: 0736-6205 cited in the application

## Description

### FIELD OF THE INVENTION

The present invention relates to methods and polynucleotide constructs for screening and obtaining high level expressing cells.

### BACKGROUND OF THE INVENTION

Production of stable mammalian cell lines that express a heterologous gene of interest begins with the transfection of a selected cell line with the heterologous gene and usually a selectable marker gene (*e.g*., neomycin^{R}). The heterologous gene and selectable gene can be cloned into and expressed from a single vector, or from two separate vectors that are co-transfected. A few days following transfection, the cells are placed in medium containing the selection agent (*e.g*., G418 for neo^{R} marker) and cultured under selection for 4-8 weeks. Once drug resistant colonies or foci have formed, these cells are isolated, expanded out and screened for expression of the desired gene product. Where the gene of interest and the selectable marker gene are cloned on separate vectors which are co-transfected into the host cell, due to the lack of physical linkage between the selectable marker gene and the product gene, survival under drug selection is not a good predictor of stable introduction and expression of the gene of interest in the host cell. The transfected cell population may contain an abundance of non-productive clones. Plating out and culturing all the transfected cells including a lot of non-producers consumes a lot of time, labor, and costly materials such as media, serum and drugs. Typically, screening of a large number of colonies or foci is required to isolate cells expressing high levels of the product of interest.

Several methods have been used to monitor gene transformation and expression. These methods include the use of reporter molecules like chloramphenicol acetyltransferase or β-galactosidase or the formation of fusion proteins with coding sequences for β-galactosidase, firefly luciferase, and bacterial luciferase. These expression assays require the cells to be fixed and incubated with exogenously added substrates or co-factors, thus destroying the cell sample, and are of limited use when cell viability is to be maintained. One method based on the co-expression of *E. coli* β-gal enzyme allows flow cytometric sorting of live cells (Nolan *et al.* PNAS USA 85: 2603-2607 (1988)). However, a hypotonic treatment is required to preload the cells with the fluorogenic substrate, and the activity must be inhibited after a specific period of time before sorting.

The advent of green fluorescent protein (GFP) as a reporter molecule provided several advantages in screening and identifying cells expressing the heterologous gene. Co-expression of GFP enables real-time analysis and sorting of transfectants by fluorescence without the requirement of additional substrates or cofactors and without destroying the cell sample. The use of GFP as a reporter molecule to monitor gene transfer has been described in various publications. Chalfie *et al.* in U.S. Patent No. 5,491,084 describe a method of selecting cells expressing a protein of interest that involves co-transfecting cells with one DNA molecule containing a sequence encoding a protein of interest, and a second DNA molecule which encodes GFP, then selecting cells which express GFP. Gubin *et al.,* in Biochem. Biophys. Res. Commun. 236: 347-350 (1997) describe transfection of CHO cells with a plasmid encoding GFP and neo to study the stable expression of GFP in the absence of selective growth conditions. Mosser *et al.,* Biotechnique 22: 150-154 (1997) describe the use of a plasmid containing a dicistronic expression cassette encoding GFP and a target gene, in a method of screening and selection of cells expressing inducible products. The target gene was linked to a controllable promoter. The plasmid incorporates a viral internal ribosome entry site (IRES) to make it possible to express a dicistronic mRNA encoding both the GFP and a protein of interest. This plasmid described by Mosser does not contain any selectable gene; the selectable gene is provided in a separate plasmid which is transfected sequentially or co-transfected with the GFP/target gene-encoding plasmid. This expression system lacks spatial and transcriptional linkage between the gene of interest, the drug selectable marker and GFP. Levenson *et al.,* Human Gene Therapy 9:1233-1236 (1998) describe retroviral vectors containing a single promoter followed by a multiple cloning site, a viral internal ribosome entry site (IRES) sequence and a selectable marker gene. The selectable markers used were those that conferred resistance to G418, puromycin, hygromycin B, histidinol D, and phelomycin, and also included GFP.

Earlier vectors incorporating an internal ribosome entry site derived from members of the picornavirus family, where the IRES is positioned between the product gene and the downstream selectable marker gene have been described (see Pelletier *et al.*, Nature 334: 320-325 (1988); Jang *et al.*, J. Virol. 63: 1651-1660 (1989); and Davies *et al.*, J. Virol. 66: 1924-1932 (1992)).

GFP has been successfully fused to other drug resistant gene products (see, *e.g.*, Bennett *et al.*, Biotechniques 24: 478-482 (1998); Primig *et al.*, Gene 215: 181-189 (1998)). Bennett *et al.,* describe a GFP fused to a zeomycin™ resistance gene (Zeo^{R}) to generate a bifunctional selectable marker for identification and selection of transfected mammalian cells. Primig describes a GFPneo vector for studying enhancers.

Lucas *et al.* in Nucleic Acids Res. 24: 1774-1779 (1996), describe expression vectors for CHO cells that express both the amplifiable selectable marker, DHFR, and a cDNA of interest, from a single primary transcript via differentially splicing. Crowley in U.S. Patent No. 5,561,053 describes a method of selecting high level producing host cells using a DNA construct containing an amplifiable selectable gene positioned within an intron, and a product gene downstream. Both the amplifiable selectable gene and the product gene are under the control of a single transcriptional regulatory region. The cells are cultured under conditions to allow gene amplification to occur. The vectors and selection methods of Lucas *et al.* and Crowley do not incorporate GFP to facilitate screening. In these and other reports, GFP was never used in conjunction with an amplifiable selectable marker in a single vector to express a protein of interest.

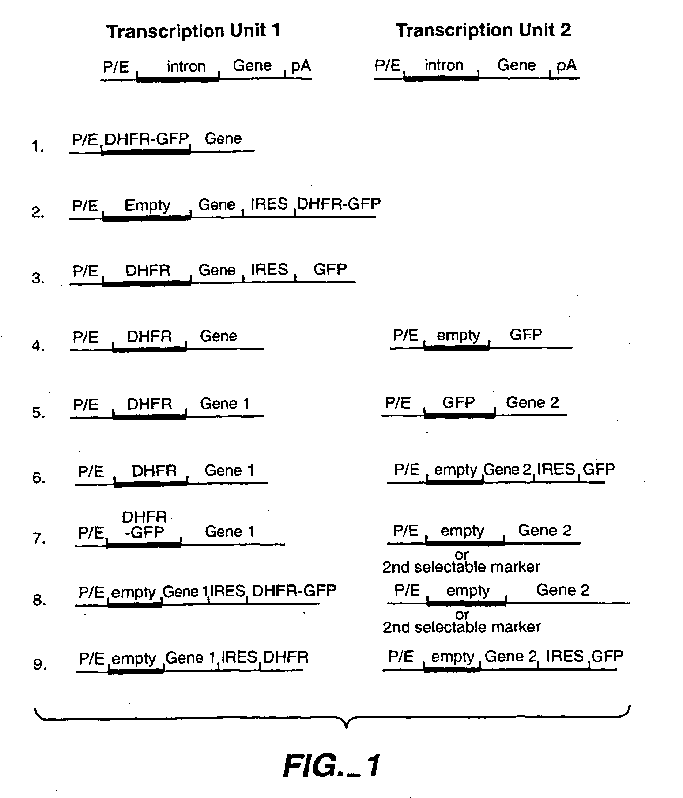

EP0711835 describes expression constructs containing a selection marker such as the dominant selection marker hygromycin B phosphotransferase (HPH). HPH is closely coupled together with the amplifiable gene DHFR to promote their co-amplification as a double-dominant marker. The DHFR/HPH fusion is operably linked to the same promoter as the gene of interest ("fremdgen"), thus maintaining a spatial and transcriptional linkage between the amplifiable gene and the gene of interest (see Figure 1).

From the above discussion, it is apparent that there is room for a better expression system that would improve the efficiency of selection and screening for recombinant cells expressing high levels of a desired product. It would be advantageous to have the gene of interest and the selectable markers in a single vector, and to be able to select for recombinant host cells which have amplified the gene of interest, to optimize the production level. Further, it it would be advantageous if the screening process enables screening of large numbers of cells at a time and is less laborious. The present invention overcomes the limitations of conventional vectors and screening methods and provides additional advantages that will be apparent from the detailed description below.

### SUMMARY OF THE INVENTION

The present invention provides vectors that allow a more efficient method of identifying and selecting for stable eukaryotic cells expressing high levels of a desired product. The present invention provides a polynucleotide comprising the following three components: a) an amplifiable selectable gene; b) a green fluorescent protein (GFP) gene; and c) at least one cloning site for insertion of a selected sequence encoding a desired product, wherein the selected sequence is operably linked to either the amplifiable selectable gene or to the GFP gene, and to a promoter. The polynucleotide comprises two transcription units.

In preferred embodiments, the amplifiable selectable gene is selected from the group of consisting of the genes encoding dihydrofolate reductase (DHFR) and glutamine synthetase. The DHFR gene is most preferred.

The GFPs suitable for use in the polynucleotides of the invention encompass wild type as well as mutant GFP. In one embodiment, the polynucleotide encodes a mutant GFP which exhibits a higher fluorescence intensity than the wild-type GFP. A specific mutant GFP is GFP-S65T having a serine to threonine substitution in amino acid 65 of the wild type protein from *Aequorea victoria.*

The polynucleotides according to the preceding embodiments further comprise an intron between the promoter and the selected sequence, the intron being defined by a 5' splice donor site and a 3' splice acceptor site. Introns suitable for use in the present vectors are preferably efficient introns that provide a splicing efficiency of at least 95%. One construct contains the amplifiable selectable-GFP fusion gene positioned within the intron, wherein both the fusion gene and the selected sequence are operably linked to one another and to the promoter present 5' of the intron.

In all the two-transcription unit constructs described herein, it will be apparent that the positions of the amplifiable selectable gene and the GFP gene can be reversed, *i*.*e*., their positions are interchangeable.

The invention further provides a polynucleotide having two transcription units, the polynucleotide comprises a first transcription unit comprising a first promoter followed by an intron and the selected sequence; and a second transcription unit comprising a second promoter and an intron 3' of the second promoter. The intron in the first transcription unit is the first intron, and the intron in the second transcription unit is the second intron; each of the first and the second introns is defined by a 5' splice donor site and a 3' splice acceptor site providing a splicing efficiency of at least 95%. In this embodiment, the amplifiable selectable gene can be positioned in the intron in the first transcription unit with both the amplifiable selectable gene and the selected sequence operably linked to the first promoter while the GFP is positioned 3' of the empty second intron and operably linked to the second promoter in the second transcription unit. Conversely, the GFP gene can be positioned in the intron in the first transcription unit, and the amplifiable selectable gene in the second transcription unit. The second transcription unit can further comprise a selected sequence operably linked to the second promoter. The selected sequence in the first transcription unit is the first selected sequence, and the selected sequence in the second transcription unit is the second selected sequence wherein the second selected sequence encodes a second desired product within the polynucleotide. In the construct of this configuration, the amplifiable selectable gene can be positioned in the first intron and the GFP gene positioned in the second intron. Alternatively, the positions of these two genes can be reversed.

In a separate embodiment of the polynucleotide which contains two transcription units; in addition to the second intron, the second transcription unit can further comprise an IRES 3' of the second selected sequence. In one polynucleotide of this configuration, the amplifiable selectable gene is positioned in the first intron and operably linked to the first promoter, and the GFP gene is positioned 3' of the IRES and operably linked to the second promoter.

In the preceding polynucleotides that contain two transcription units and a promoter in each unit, the same or different type of promoter can be used as the first promoter and the second promoter. Polynucleotides are provided wherein one or more of the promoters in the transcription units is an inducible promoter. In a preferred embodiment, the promoter in the transcription unit or units is the CMV IE or the SV40 promoter.

In preferred embodiments, the polynucleotides of the invention will contain a selected sequence encoding a protein selected from the group consisting of cytokines, lymphokines, enzymes, antibodies, and receptors. In specific embodiments, the selected sequence encodes neuronotrophin-3, deoxyribonuclease, vascular endothelial growth factor, immunoglobulin and Her2 cell surface protein.

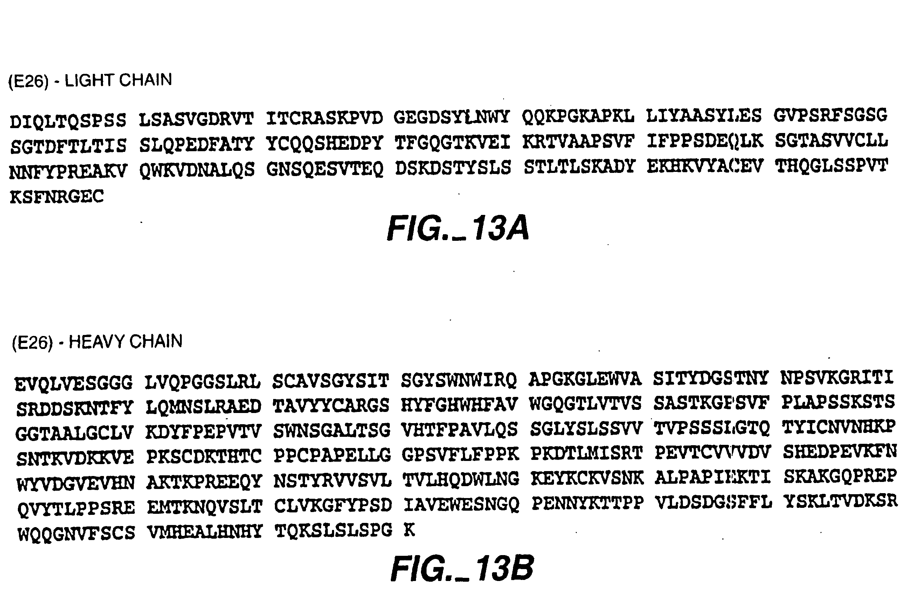

Where the desired product is a multichain (*e.g.*, a heterodimeric) receptor, the first selected sequence can encode one polypeptide chain of the multichain receptor, and the second selected sequence can encode a second polypeptide chain of the receptor. Where the multichain protein is an immunoglobulin, the first selected sequence can encode the immunoglobulin heavy (H) chain and the second selected sequence encodes the light (L) chain. In preferred embodiments, the immunoglobulin expressed from the polynucleotide is a humanized immunoglobulin. The invention provides a polynucleotide in which the selected sequences encode a anti-IgE antibody. In one specific embodiment, the anti-IgE is the full length E26, humanized antibody having the amino acid sequence of SEQ ID NO. 1 (H chain) and SEQ ID NO. 2 (L chain) shown in Fig. 13A and Fig. 13B, respectively.

A polynucleotide of the invention that replicates in a eukaryotic host cell is also provided.

The invention also provides host cells, both bacterial and eukaryotic host cells containing the polynucleotides of the invention. A preferred mammalian cell is a Chinese Hamster Ovary (CHO) cell. Where the amplifiable selectable gene present in the constructs is the DHFR gene, the preferred host cell is a CHO cell having a DHFR⁻ phenotype. The invention provides host cells producing a desired product selected from the group consisting of neuronotrophin-3, deoxyribonuclease, vascular endothelial growth factor, Her2, and anti-IgE antibody.

Another aspect of the invention is method of producing a desired product by introducing a polynucleotide of the invention into a suitable eukaryotic cell, culturing the resultant eukaryotic cell under conditions so as to express the desired product, and recovering the desired product. Preferably, the desired product is secreted from the cell where it can be recovered from the culture medium.

A cell expressing a desired product may be obtained by introducing a polynucleotide of the invention into a population of eukaryotic cells and isolating the resultant cells that express the green fluorescent gene and the amplifiable selectable gene, expression of these genes indicative of the cell also expressing the desired product. Cells expressing the green fluorescent protein can be isolated by sorting using fluorescence activated cell sorter (FACS) to sort and clone high fluorescent cells which are preferably the brightest 1%-10% of fluorescent cells within the sorted population. The cells can be subjected to repeated rounds of sorting to enrich for the brightest fluorescent cells. The cells are cultured for a period of time, preferably about two weeks, between each round of sorting and cloning. Preferably, the cells are cultured in selection medium during the period of time. Preferably, the high fluorescent cells are cultured in selection medium that contains an appropriate amplifying agent, to amplify at least the amplifiable selectable gene and the selected sequence. Gene amplification can be achieved by subjecting the cells to incremental amounts of the amplifying agent in culture. In a preferred embodiment, the amplifiable selectable gene is DHFR and the amplifying agent is methotrexate. After the cells have been subjected to gene amplification by culturing in the presence of the amplifying agent, the cells are further analyzed to confirm expression of the desired protein and to identify and isolate the high producing cells. In one embodiment, expression of the desired protein is determined by analyzing the cells for RNA encoding the desired product, using the technique of RT-PCR, the amount of specific RNA indicative of the level of production of the desired product.

### BRIEF DESCRIPTION OF THE DRAWINGS

Figure 1 schematically shows 9 exemplary construct designs. Gene refers to gene of interest; empty means intron without an inserted gene; DHFR·GFP refers to the fusion gene.
Figure 2 shows the translation products and their relative amounts resulting from different transcripts, spliced and unspliced. Figures 2A, 2B, and 2C correspond to configurations 1, 3, and 4, respectively, in Figure 1. Goi refers to the gene of interest; TU, transcription unit; T1-4 refer to the different transcripts from the indicated region of the construct.
Figure 3 schematically shows intron and IRES combinations in a vector having a single transcription unit for expression of the gene of interest. For GFP selection, the GFP gene can be intronic (transcriptionally linked), after the IRES sequence (translationally linked), or expressed as a fusion protein linked to a selectable marker and located in the intron or after the IRES sequence.
Figure 4 shows intron and IRES combinations in multiple transcription unit configurations for expression of the exemplary E26 antibody heavy and light chain to form the complete E26 antibody.
Figure 5 shows an exemplary intronic DHFR intron vector-construct, pSV15.ID.LLn, as described in Example 1.
Figure 6 shows an example of the two transcription units vector for expressing VEGF; see Figure 1, configuration 4.
Figure 7 shows that GFP protein in cell lysates measured by ELISA correlated with GFP fluorescence measured by FACS in 18 GFP expressing clones (correlation coefficient = 0.99, p <0.0001). Error bars were standard deviations from at least two ELISA data points.
Figure 8A shows NT3 productivity vs GFP. fluorescence in 17 NT3-GFP producing clones (correlation coefficient = 0.68, p = 0.0018); Figure 8B shows relative NT3 RNA versus NT3 productivity (correlation coefficient = 0.89, p < 0.0001).
Figure 9A shows DNase productivity vs GFP fluorescence in 15 DNase-GFP producing clones (correlation coefficicnt = 0.52, p < 0.048). Error bars were standard deviations of at least 3 ELISA data points. Figure 9B shows relative DNase RNA versus DNase productivity (correlation coefficient = 0.90, p < 0.0001). Error bars were standard deviations of two RT-PCR measurements.

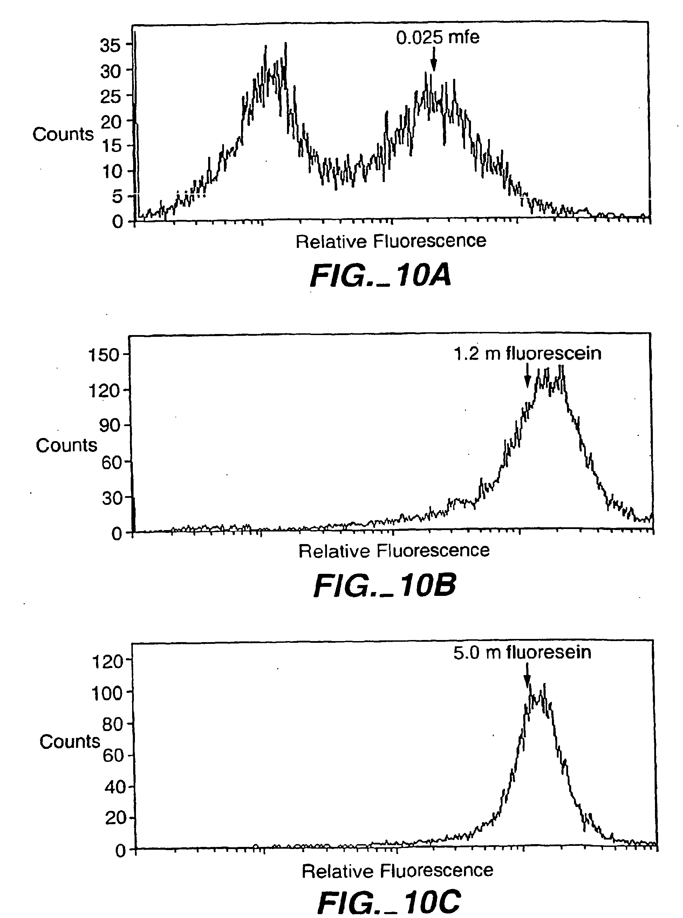

Figure 10 shows the flow cytometry profiles of CHO cells expressing VEGF and GFP. Figure 10A shows the fluorescence profile of cells two weeks after transfection just before the first sort. The fluorescence intensity of the right peak is 0.025 mfe. The background fluorescence of the non-transfected cells was 0.0005 mfe. Figure 10B shows the fluorescence profile of cells just before the third sort. The mean fluorescence intensity was 1.2 mfe. These cells were obtained by collecting 35,000 cells with the top 2.5% fluorescence at the first sort and 50,000 cells with the top 1.5% fluorescence at the second sort. Cells were grown for two weeks between sorts. Cells with the top 0.5% fluorescence were cloned by FACS. Figure 10C shows the fluorescence profile of the clone with the highest fluorescence. The fluorescence intensity was 5.0 mfe.

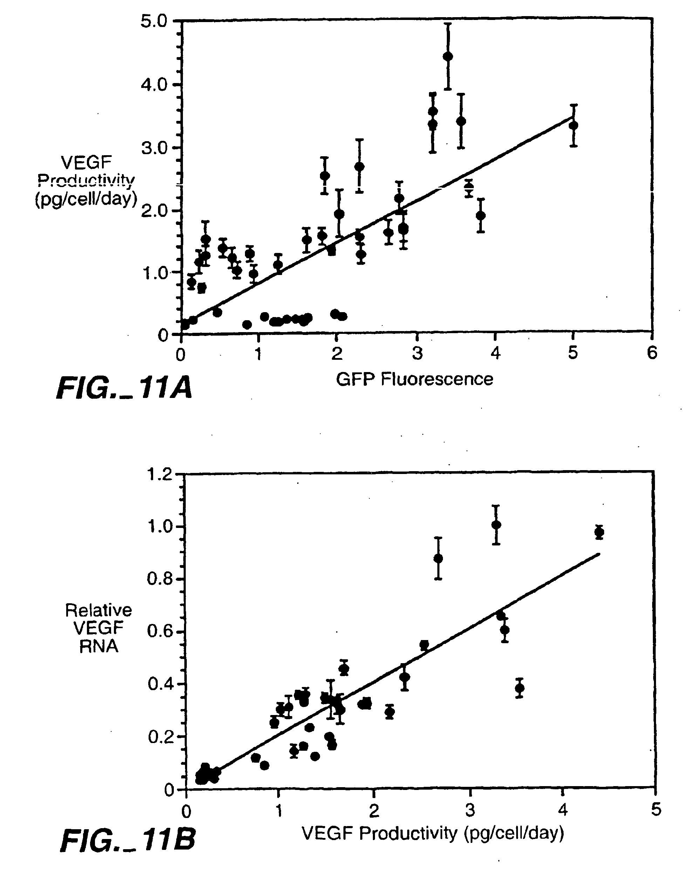

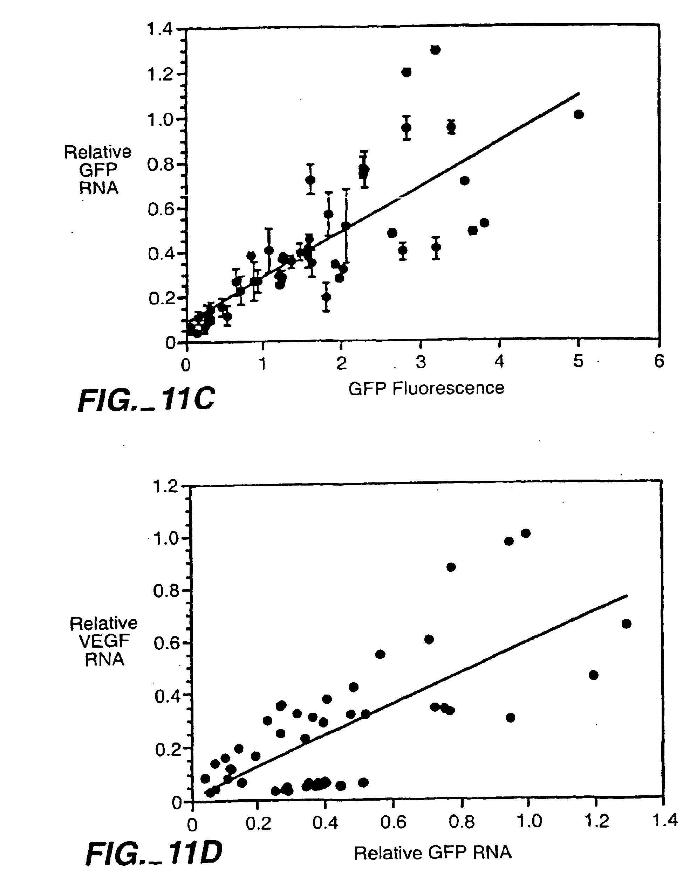

Figure 11A shows VEGF productivity versus GFP fluorescence in 48 VEGF-GFP producing clones (correlation coefficient = 0.70, p < 0.0001). Concentrations of VEGF were average of at least 3 data points. Error bars were standard deviations. Figure 11B shows relative VEGF RNA versus VEGF productivity (correlation coefficient = 0.90, p < 0.0001). Figure 11C shows relative GFP RNA versus GFP fluorescence (correlation coefficient = 0.78, p < 0.0001). Figure 11D shows relative VEGF RNA versus relative GFP RNA (correlation coefficient = 0.71, p < 0.0001). Error bars were standard deviations of two RT-PCR measurements. The amount of VEGF or GFP RNA was normalized to the RNA in the clone with the highest fluorescence.
Figure 12 shows a comparison of VEGF productivity in the top 5 producing clones obtained by either random picking and screening VEGF clones (open square) or by FACS sorting based on GFP fluorescence intensity and cloning of VEGF-GFP producing cells (open circle); and in the top 5 populations in MTX obtained by either random picking VEGF producing populations (3 from 25 nM, 1 from 50 nM and 1 from 100 nM) (closed square) or by fluorescence microscopy screening of VEGF-GFP producing cells (2 from 25 nM and 3 from 50 nM) (closed circle).
Figure 13 shows the amino acid sequences of the full length heavy (Fig. 13A; SEQ ID NO. 1) and light chains (Fig. 13B; SEQ ID NO. 2) of the anti-IgE antibody, E26.

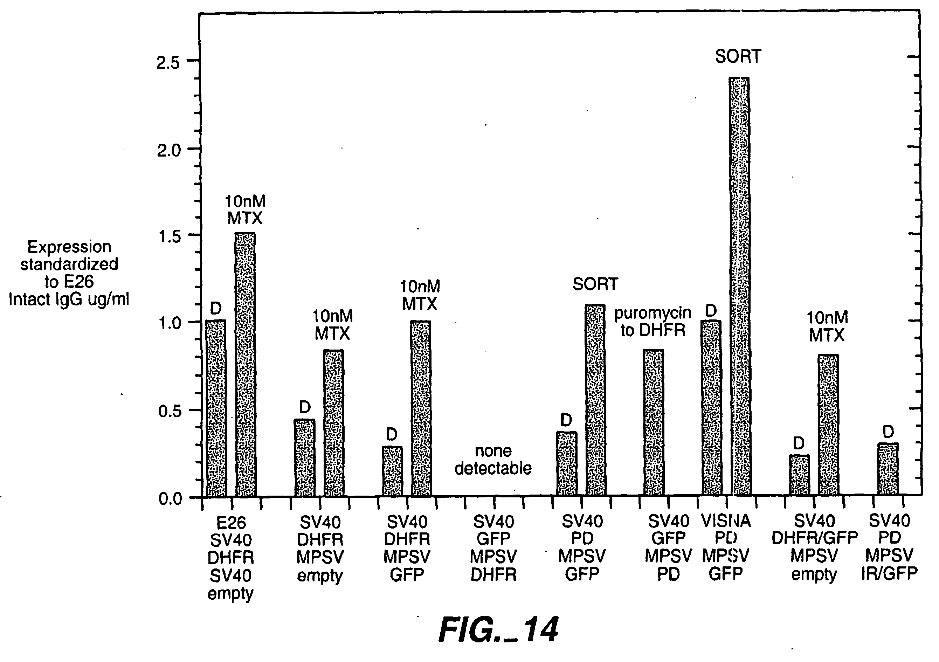

Figure 14 shows E26 antibody expression levels from different GFP configurations. The labeling under each bar of the graph indicates in order of 5' to 3', the promoter used to transcribe the H chain (SV40 or MPSV=Myeloproliferative sarcoma virus promoter and enhancer or VISNA=a lentivirus P/E), the selectable marker in the 1^{st} intron (DHFR, GFP, PD=puromycin/DHFR fusion, DHFR/GFP= fusion), the promoter used to transcribe the L chain, and the marker present in the 2^{nd} intron of the 2^{nd} transcription unit: Empty refers to empty intron; IR/GFP refers to IRES followed by GFP gene with the 2^{nd} intron empty.
Figure 15 shows the mean GFP values of cells expressing E26 from vectors with different configurations of GFP.

### DESCRIPTION OF THE PREFERRED EMBODIMENTS

This invention provides vectors that include the amplifiable selectable gene, the GFP gene and a sequence encoding a desired product, wherein these elements are present in a single vector and wherein two or more of these elements are under the transcriptional control of the same promoter. Expression of GFP together with an amplifiable selectable marker provides a more efficient method of selecting for and identifying eukaryotic cells expressing a heterologous gene at high levels. The amplifiable selectable marker not only allows selection of stable transfected mammalian cell lines but allows amplification of the heterologous gene of interest. As demonstrated below, the vectors and methods of the invention achieved high level expression of proteins of varying characteristics. These proteins included enzymes, antibodies, secreted proteins, cell surface receptors as well as novel proteins of as yet unknown function, the open reading frames of which were prepared or pieced together from sequence databases. Thus, the vectors of the invention are also useful in high throughput screening of genomics.

GFP fluorescence provides a noninvasive technique for earlier and faster screening of transfected cells. The small size of GFP keeps the overall size of the vectors small, allowing for high transformation and transfection efficiencies. Green fluorescent protein does not require any substrates, co-factors or enzymes for its fluorescence, making the protein unique in that it can be detected in real time. The detection of intracellular GFP requires only irradiation by near UV or blue light. Since GFP does not require any staining techniques, it is a better alternative than conventional enzyme and antibody based methods for monitoring gene expression in single cells. Expression of GFP does not appear to interfere with cell growth or function. Cells expressing GFP can be separated out by fluorescence-activated cell sorting. The FACS can sort more than 2000 cells/sec, between about 3,000-10,000 cells/see, making it possible to screen a large number of cells to find high producing clones. It greatly reduces the amount of work and makes it possible to obtain high producing clones when an ELISA for the desired protein is not available.

It was believed that closer spatial as well as transcriptional and translational linkage between the amplifiable selectable marker gene and the gene of interest, would enhance the probability of co-amplification of both genes under selection pressure. However, initially, the integrity of the integrated expression vector and of the transcriptional linkage between the product gene of interest and the amplifiable gene as well as the GFP reporter gene upon amplification, was not predictable. It was possible that the gene of interest and/or the GFP gene may be deleted during amplification, as was previously reported with the DHFR gene (Kaufman *et al.* Mol. & Cell. Biol. 12: 1069-1076 (1981); Kaufman and Sharp, J. Mol. Biol. 159:601-621 (1982). Surprisingly, as demonstrated in the Examples, use of the polynucleotides of the invention demonstrated a good correlation between expression of the desired protein (by RNA and product titer) and GFP fluorescence, demonstrating a good co-expression efficiency of two linked transcription units and no apparent loss of these genes during amplification.

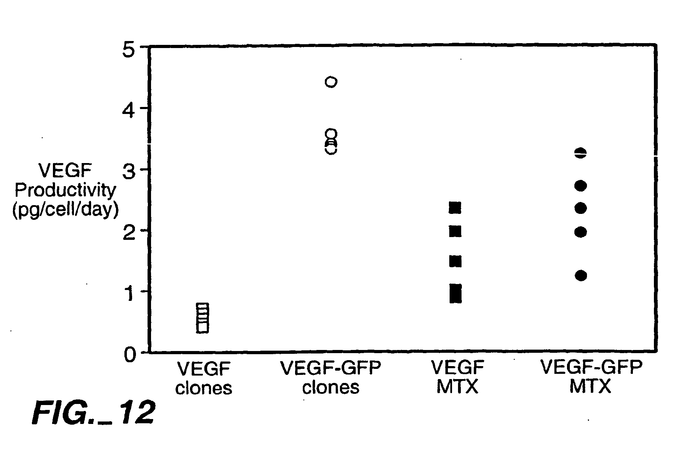

The invention also showed that sorting cells according to the intensity of GFP fluorescence using the FACS increased the chance of obtaining high producing clones. Indeed, higher producing clones were obtained by FACS sorting than by randomly picking 144 clones by hand and screening by ELISA (see Fig. 12). FACS sorting would be particularly useful to obtain high producing clones for molecules which arc difficult to express. The experiments herein also show that clones obtained by FACS sorting could be amplified with MTX to obtain higher producing clones.

Additionally, the invention demonstrated that the amount of RNA of the desired protein correlated very well with the product titer und therefore, high producing clones can be obtained by measuring the amount of RNA of the desired protein in the highly fluorescent clones. This is very useful when secreted proteins of unknown function are expressed from the DNA sequence data base, for screening for biological activities.

### Definitions

A "polynucleotide" as used herein, refers to a non-naturally occurring, recombinantly produced, polymeric form of nucleotides of any length, either ribonucleotides or deoxyribonuclcotides, or analogs thereof. This term refers to the primary structure of the molecule, and thus includes double- and single stranded DNA, as well as double- and single-stranded RNA. It also includes modified polynucleotides such as methylated and/or capped polynucleotides. The polynucleotide can either be an isolate, or integrated in another nucleic acid molecule *e*.*g*. in an expression vector or the chromosome of an eukaryotic host cell. Polynucleotide includes self-replicating plasmids. The terms "construct" and "vector" are used interchangeably with "polynucleotide" herein. Vector includes shuttle and expression vectors. Typically, the plasmid construct will also include an origin of replication (*e*.*g*., the ColE1 origin of replication) and a selectable marker (*e.g*., ampicillin or tetracycline resistance), for replication and selection, respectively, of the plasmids in bacteria. A polynucleotide or construct includes but does not have to be, an expression vector. An "expression vector" refers to a construct that contains the necessary regulatory elements for expression of at least the amplifiable selectable gene, GFP gene and selected sequence in the host cell.

As used herein, a "fluorescent protein" refers to any protein that emits sufficient fluorescence to enable fluorescence detection of the protein intracellularly by, *e.g*., fluorescence microscopy or flow cytometry. Preferably, host cells expressing fluorescent proteins can be detected using a fluorescence-activated cell sorter (FACS). Examples of fluorescent proteins include green, cyan, blue, yellow as well as other fluorescent proteins from the coclenterate sub-phylum Cnidaria. The fluorescent protein encoding sequences can be native (wild-type) genes, or variants of the genes which are synthetic prepared such as by genetic engineering. A preferred fluorescent protein is green fluorescent protein (GFP), preferably from *Aequorea victoria*. In one embodiment, the *Aequorea* GFP mutant, S65T, (described below) is used.

Two well characterized GFPs arc from the jellyfish, *Aequorea victoria*, and a sea pansy, *Renilla reniformis*. *Aequorea* and *Renilla* GFPs each transmute blue chemiluminescence from a distinct primary photoprotein into green fluorescence. *Aequorea* GFP is a protein of 238 amino acid residues. The protein is maximally excited with blue light with a bigger absorbance peak at 395 nm and a smaller peak at 475 nm, and emits green light at 508-509 nm. The mature purified protein is highly stable, remaining fluorescent up to 65°C, pH11, 1% SDS or 6M guanidinum chloride, and resisting most proteases for may hours. *Renilla* GFP is an even more stable protein than *Aequorea* GFP; it shows a single absorption peak at 498 nm with an emission peak at 509 nm. For a review of the properties of *Aequorea* and *Renilla* GFPs, see, *e.g.*, Chalfie *et al.*, Science 263: 802-805 (1994); and Cubitt *et al.*, Trends Biochem. Sci. 20: 448-455 (1995). GFP can fluoresce in both transformed prokaryotic and eukaryotic cells.

The invention encompasses the use of any form or derivative of GFP that emits sufficient fluorescence to enable fluorescence detection of intracellular GFP by flow cytometry using a fluorescence-activated cell sorter (FACS), or by fluorescence microscopy. GFP usable in the invention include wild-type as well as naturally occurring (by spontaneous mutation) or recombinantly engineered mutants and variants, truncated versions and fragments, functional equivalents, derivatives, homologs and fusions, of the naturally occurring or wild-type proteins. A range of mutations in and around the chromophore structure of GFP (around amino acids 64-68) have been described. These mutations result in modifications of the spectral properties, the speed of chromophore formation, the extinction coefficient, and the physical characteristics of the GFP. These forms of GFP may have altered excitation and emission spectra as compared to the wild-type GFP, or may exhibit greater stability. The mutant GFPs may fluoresce with increased intensity or with visibly distinct colors than the wild-type protein, *e.g*., blue, yellow or red-shifted fluorescent proteins, the DNA containing these genes of which are available commercially (Clontech, Palo Alto, CA; Quantum Biotechnologies, Montreal, Canada). Mutants with increased fluorescence over the wild-type GFP provide a much more sensitive detection system. Mutants may have a single excitation peak as opposed to 2 peaks characteristic of the native protein, may be resistant to photobleaching or may exhibit more rapid oxidation to fluorophore. For example, the *Aequorea* GFP mutant, S65T (Heim *et al.* Nature 373: 663-664 (1995)), in which Ser65 has been replaced by Thr, offers several advantages over the wild-type GFP in that the mutant provides six-fold greater brightness than wild-type, faster fluorophore formation, no photoisomerization and only very slow photobleaching. Modifications of Ser65 to Thr or Cys result in GFPs that continue to emit maximally at -509 nm but which have a single excitation peak red-shifted to 488 nm and 473 nm respectively. This has several advantages in that it brings the excitation peaks more in line with those already used with fluorescent microscopes and fluorescence-activated cell sorters (FACS) for FITC. Furthermore, chromophore formation of these mutants is more rapid and the extinction coefficient is greater than that of wtGFP (wild-type GFP), which results in a stronger fluorescent signal (Heim *et al.,* 1995, supra). Other GFP mutants have codons optimized for mammalian cell expression as well as exhibiting greater fluorescence than the original GFP gene (see Bennet (1998), infra; Crameri *et al.* Nature Biotechnol. 14:315-319 (1996)). "Humanized" or otherwise modified versions of GFP, including base substitution to change codon usage, that favor high level expression in mammalian cells, are suitable for use in the constructs of the invention (see, *e.g*., Hauswirth *et al.,* U.S. Patent No. 5,874,304; Haas *et al.* U.S. Patent No. 5,795,737). GFP mutants that will fluoresce and be detected by illumination with white light are described in WO 9821355. Still other mutant GFPs are described in U.S. Patent No. 5,804,387 (Cormack *et al*.) and WO 9742320 (Gaitanaris *et al*.). GFP has been functionally expressed as a fusion protein (see, *e.g*., Marshall *et al.* Neuron 14: 211-215 (1995); Olson *et al.* J. Cell. Biol. 130:639-650 (1995); Bennett *et al.,* Biotechniques 24: 478-482 (1998)). The GFP fusion proteins useful in the present invention include fusions with the amplifiable selectable marker that confer the combined properties of amplifiable selection and fluorescence of the individual proteins. An example of such a fusion protein is a GFP-DHFR fusion protein. Therefore, "green fluorescent protein gene" as used herein, includes sequences encoding any of the preceding polypeptides.

A "selectable marker gene" is a gene that allows cells carrying the gene to be specifically selected for or against, in the presence of a corresponding selection agent. By way of illustration, an antibiotic resistance gene can be used as a positive selectable marker gene that allows the host cell transformed with the gene to be positively selected for in the presence of the corresponding antibiotic; a non-transformed host cell would not be capable of growth or survival under the selection culture conditions. Selectable markers can be positive, negative or bifunctional. Positive selectable markers allow selection for cells carrying the marker, whereas negative selection markers allow cells carrying the marker to be selectively eliminated. Typically, a selectable marker gene will confer resistance to a drug or compensate for a metabolic or catabolic defect in the host cell. The selectable marker genes used herein including the amplifiable selectable genes, will include variants, fragments, functional equivalents, derivatives, homologs and fusions of the native selectable marker gene so long as the encoded product retains the selectable property. Useful derivatives generally have substantial sequence similarity (at the amino acid level) in regions or domains of the selectable marker associated with the selectable property. A variety of marker genes have been described, including bifunctional (*i.e*., positive/negative) markers (see *e.g.,* WO 92/08796, published 29 May 1992, and WO 94/28143, published 8 Dec. For example, selectable genes commonly used with eukaryotic cells include the genes for aminoglycoside phosphotransferase (APH), hygromycin phosphotransferase (hyg), dihydrofolate reductase (DHFR), thymidine kinase (tk), glutamine synthetase, asparagine synthetase, and genes encoding resistance to neomycin (G418), puromycin, histidinol D, bleomycin and phleomycin.

An "amplifiable selectable gene" has the properties of a selectable marker gene as defined above, but additionally can be amplified (*i.e.,* additional copies of the gene are generated which survive in intrachromosomal or extrachromosomal form) under appropriate conditions. The amplifiable selectable gene usually encodes an enzyme which is required for growth of eukaryotic cells under those conditions. For example, the amplifiable selectable gene may encode DHFR (dihydrofolate reductase) which gene is amplified when a host cell transfected therewith is grown in the presence of the selective agent, methotrexate (Mtx). The exemplary selectable genes in Table 1 below are also amplifiable selectable genes. An example of a selectable gene which is generally not considered to be an amplifiable gene is the neomycin resistance gene (Cepko *et al., supra*).

For references directed to co-transfection of a gene together with a genetic marker that allows for selection and subsequent amplification, see, *e.g*., Kaufman in Genetic Engineering, ed. J. Setlow (Plenum Press, New York), Vol. 9 (1987); Kaufman and Sharp, J. Mol. Biol., 159:601 (1982); Ringold *et al.,* J. Mol. Appl. Genet., 1:165-175 (1981); Kaufman *et al*., Mol. Cell Biol., 5:1750-1759 (1985); Kaetzel and Nilson, J. Biol. Chem., 263:6244-6251 (1988); Hung *et al.,* Proc. Natl. Acad. Sci. USA, 83:261-264 (1986); Kaufman *et al.,* EMBO J., 6:87-93 (1987); Johnston and Kucey, Science, 242:1551-1554 (1988); Urlaub *et al.,* Cell, 33:405-412 (1983). For a review of the amplifiable selectable genes listed in Table 1, see Kaufman, Methods in Enzymology, 185: 537-566 (1990).

**TABLE 1 Amplifiable Selectable Genes and their Selection Agents**

| Selection Agent | Selectable Gene |
|---|---|
| Methotrexate | Dihydrofolate reductase |
| Cadmium | Metallothionein |
| PALA | CAD |
| Xyl-A-or adenosine and 2'-deoxycoformycin | Adenosine deaminase |
| Adenine, azaserine, and coformycin | Adenylate deaminase |
| 6-Azauridine, pyrazofuran | UMP Synthetase |
| Mycophenolic acid | IMP 5'-dehydrogenase |
| Mycophenolic acid with limiting xanthine | Xanthine-guanine phosphoribosyltransferase |
| Hypoxanthine, aminopterin, and thymidine (HAT) | Mutant HGPRTase or mutant thymidine kinase |
| 5-Fluorodeoxyuridine | Thymidylate synthetase |
| Multiple drugs *e.g.* adriamycin, vincristine or colchicine | P-glycoprotein 170 |
| Aphidicolin | Ribonucleotide reductase |
| Methionine sulfoximine | Glutamine synthetase |
| β-Aspartyl hydroxamate or Albizziin | Asparagine synthetase |
| Canavanine | Arginosuccinate synthetase |
| α-Difluoromethylornithine | Ornithine decarboxylase |
| Compactin | HMG-CoA reductase |
| Tunicamycin | *N*-Acetylglucosaminyl transferase |
| Borrelidin | Threonyl-tRNA synthetase |
| Ouabain | Na⁺K⁺-ATPase |

A preferred amplifiable selectable gene is the gene encoding dihydrofolate reductase (DHFR) which is necessary for the biosynthesis of purines. Cells lacking the DHFR gene will not grow on medium lacking purines. The DHFR gene is therefore useful as a dominant selectable marker to select and amplify genes in such cells growing in medium lacking purines. The selection agent used in conjunction with a DHFR gene is methotrexate (Mtx).

As used herein, "selection medium" refers to nutrient solution used for growing eukaryotic cells which contain and express the selectable gene and therefore includes a "selection agent". Commercially available media such as Ham's F10 (Sigma), Minimal Essential Medium ([MEM], Sigma), RPMI-1640 (Sigma), and Dulbecco's Modified Eagle's Medium ([DMEM], Sigma) are exemplary nutrient solutions. In addition, any of the media described in Ham and Wallace, Meth. Enz., 58:44 (1979), Barnes and Sato, Anal. Biochem., 102:255 (1980), U.S. Patent Nos. 4,767,704; 4,657,866; 4,927,762; or 4,560,655; WO 90/03430; WO 87/00195; U.S. Patent Re. 30,985; or U.S. Patent No. 5,122,469, may be used as culture media. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics (such as Gentamycin™ drug), trace elements (defined as inorganic compounds usually present at final concentrations in the micromolar range), and glucose or an equivalent energy source. The media is frequently supplemented with serum, *e.g.,* fetal calf or horse serum, as a source of hormones, growth factors and other elements. Any other necessary supplements may also be included at appropriate concentrations that would be known to those skilled in the art.

The term "selection agent" refers to a substance that interferes with the growth or survival of a host cell that is deficient in a particular selectable gene. Examples of selection agents are presented in Table 1 above. The selection agent preferably comprises an "amplifying agent" which is defined for purposes herein as an agent for amplifying copies of the amplifiable gene. The selection agent can also be the amplifying agent if the selectable marker gene relied on is an amplifiable selectable marker. For example, Mtx is a selection agent useful for the amplification of the DHFR gene. See Table 1 for examples of amplifying agents.

"Selected sequence" or "product gene" or "gene of interest" have the same meaning herein and refer to a polynucleotide sequence of any length that encodes a product of interest. Typically, the selected sequence will be in the range of from 1-20 kilobases (kb) in length, preferably from 1-5 kb. The gene of interest will be a heterologous gene with respect to the host cell. The selected sequence can be a full length or a truncated gene, a fusion or tagged gene, and can be a cDNA, a genomic DNA, or a DNA fragment, preferably, a cDNA. The selected sequence can be the native sequence i.e., naturally occurring form(s), or can be mutated or otherwise modified as desired. These modifications include humanization, codon replacement to optimize codon usage in the selected host cell or tagging. The selected sequence can encode a secreted, cytoplasmic, nuclear, membrane bound or cell surface polypeptide. Expression of the selected sequence should not be detrimental to the host cell or compromise cell viability. The "desired product" includes proteins, polypeptides and fragments thereof, peptides, and antisense RNA, which are capable of being expressed in the selected eukaryotic host cell. The proteins can be hormones, cytokines and lymphokines, antibodies, receptors, adhesion molecules, enzymes, and fragments thereof. The desired proteins can serve as agonist or antagonist, and/or have therapeutic or diagnostic uses. The present polynucleotides are most suitable for expression of desired products of mammalian origin although microbial and yeast products can also be produced.

The terms "polypeptide" and "protein" are used interchangeably to refer to polymers of amino acids of any length. These terms also include proteins that are post-translationally modified through reactions that include glycosylation, acetylation and phosphorylation. The term "peptide" refers to shorter stretches of amino acids, generally less than about 30 amino acids.

The term "antibody" or "immunoglobulin" as used herein includes monoclonal antibodies, polyclonal antibodies, multispecific antibodies (*e.g.,* bispecific antibodies), single chain antibodies including sFv dimers, antibody fragments (*e.g*., Fab, Fab', F(ab')₂, Fv) and diabodies so long as they exhibit the desired biological activity. The antibodies can be of any species and include humanized antibodies. "Humanized" forms of non-human (*e.g.* murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. For the most part, humanized antibodies are human immunoglobulins (recipient antibody) in which residues from a complementary determining region (CDR) of the recipient are replaced by residues from a CDR of an antibody from a non-human species (donor antibody) such as mouse, rat or rabbit, having the desired specificity, affinity or function. In some instances, Fv framework residues of the human immunoglobulin are replaced by corresponding non-human residues. Furthermore, humanized antibody may comprise residues which are found neither in the recipient antibody nor in the imported CDR or framework sequences. These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally will also comprise at least a portion of an immunoglobulin constant region (Fc), typically that of a human immunoglobulin. For further details, see: Jones *et al*., *Nature* 321, 522-525. (1986); Reichmann *et al*., *Nature* 332, 323-329 (1998) and Presta. *Curr. Op. Struct. Biol.* 2, 593-596 (1992).

"Regulatory elements" as used herein, refer to nucleotide sequences present in cis, necessary for transcription and translation of GFP gene, the amplifiable selectable gene, and the selected sequence of interest, into polypeptides. The transcriptional regulatory elements normally comprise a promoter 5' of the gene sequence to be expressed, transcriptional initiation and termination sites, and polyadenylation signal sequence. The term "transcriptional initiation site" refers to the nucleic acid in the construct corresponding to the first nucleic acid incorporated into the primary transcript, *i.e*., the mRNA precursor; the transcriptional initiation site may overlap with the promoter sequences. The term "transcriptional termination site" refers to a nucleotide sequence normally represented at the 3' end of a gene of interest or the stretch of sequences to be transcribed, that causes RNA polymerase to terminate transcription. The polyadenylation signal sequence, or poly-A addition signal provides the signal for the cleavage at a specific site at the 3' end of eukaryotic mRNA and the post-transcriptional addition in the nucleus of a sequence of about 100-200 adenine nucleotides (polyA tail) to the cleaved 3' end. The polyadenylation signal sequence includes the sequence AATAAA located at about 10-30 nucleotides upstream from the site of cleavage, plus a downstream sequence.

The promoter can be constitutive or inducible. An enhancer (*i.e.*, a *cis*-acting DNA element that acts on a promoter to increase transcription) may be necessary to function in conjunction with the promoter to increase the level of expression obtained with a promoter alone, and may be included as a transcriptional regulatory element. Often, the polynucleotide segment containing the promoter will include the enhancer sequences as well (*e.g*., CMV IE P/E; SV40 P/E; MPSV P/E). Splice signals may be included where necessary to obtain spliced transcripts. To produce a secreted polypeptide, the selected sequence will generally include a signal sequence encoding a leader peptide that directs the newly synthesized polypeptide to and through the ER membrane where the polypeptide can be routed for secretion. The leader peptide is often but not universally at the amino terminus of a secreted protein and is cleaved off by signal peptidases after the protein crosses the ER membrane. The selected sequence will generally, but not necessarily, include its own signal sequence. Where the native signal sequence is absent, a heterologous signal sequence can be fused to the selected sequence. Numerous signal sequences are known in the art a and available from sequence databases such as GenBank and EMBL. Translational regulatory elements include a translational initiation site (AUG), stop codon and poly A signal for each individual polypeptide to be expressed. An internal ribosome entry site (IRES) is included in some constructs. IRES is defined below.

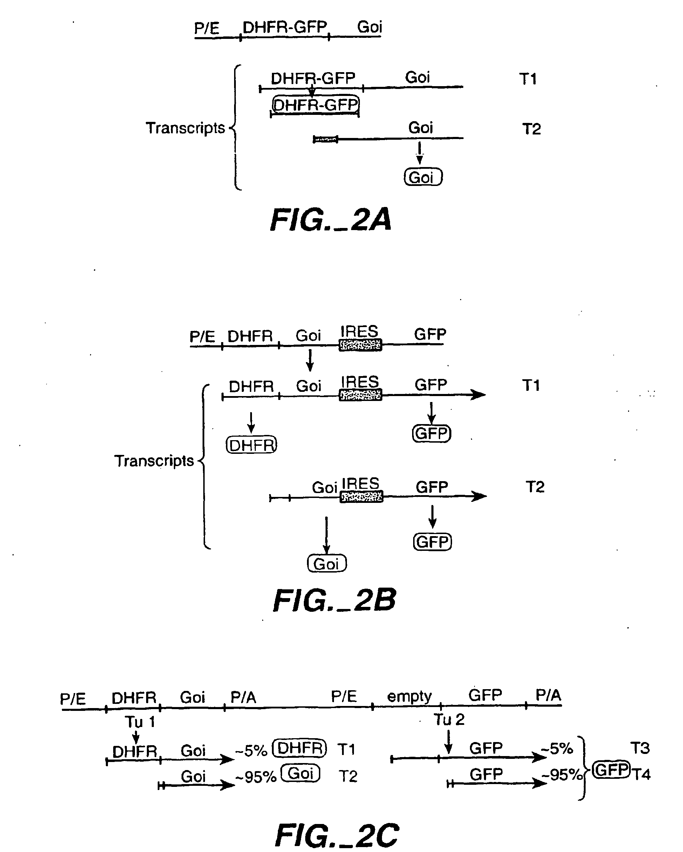

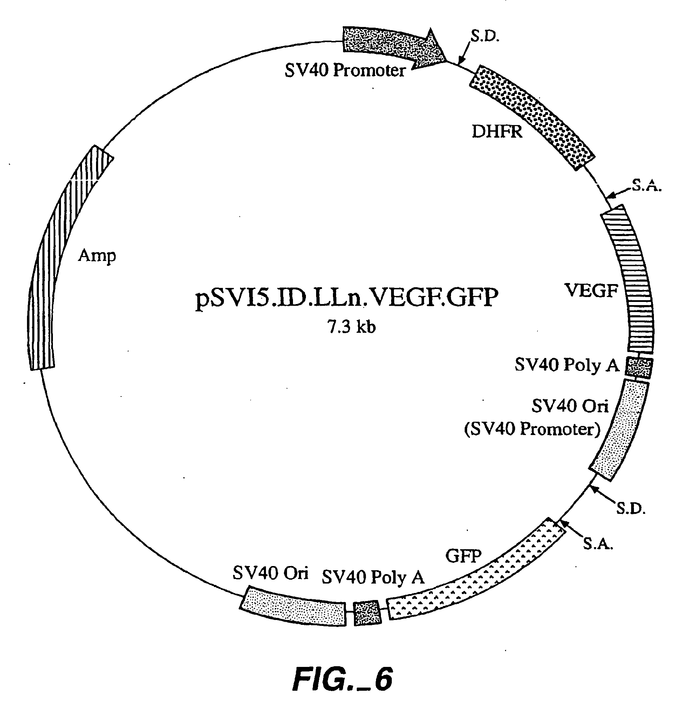

An "transcription unit" defines a region within a construct that contains one or more genes to be transcribed, wherein the genes contained within that segment are operably linked to each other and transcribed from a single promoter, and as a result, the different genes are at least transcriptionally linked. More than one protein or product can be transcribed and expressed from each transcription unit. Each transcription unit will comprise the regulatory elements necessary for the transcription and translation of any of the selected sequence, GFP and amplifiable selectable marker genes that are contained within the unit, as well as any additional selectable marker genes that may be operably linked to one of these three components in the same transcription unit. As an illustration, Figure 6 shows a construct comprising two separate transcription units; DHFR and the desired protein are expressed from the first transcription unit and GFP is expressed from the second transcription unit. In the first transcription unit, DHFR gene and the selected sequence encoding the desired product are operably linked to each other and to the SV40 promoter. Transcription proceeds through the DHFR and the selected sequence to the polyA signal, producing a full length primary transcript that encodes both genes. Each of the genes in the transcription unit has its own translation initiation codon, ATG. The second transcription unit comprises the GFP gene and regulatory elements necessary for GFP expression. The GFP gene is independently transcribed from a second SV40 promoter within the construct. Each transcription unit will contain its own promoter but the type of promoter can be the same or different. In the example depicted in Figure 2, the first and second transcription units use the same type of promoter, SV40 promoter in this case.

A "promoter" refers to a polynucleotide sequence that controls transcription of a gene or sequence to which it is operably linked. A promoter includes signals for RNA polymerase binding and transcription initiation. The promoters used will be functional in the cell type of the host cell in which expression of the selected sequence is contemplated. A large number of promoters including constitutive, inducible and repressible promoters from a variety of different sources, are well known in the art (and identified in databases such as GenBank) and are available as or within cloned polynucleotides (from, *e.g*., depositiories such as ATCC as well as other commercial or individual sources). With inducible promoters, the activity of the promoter increases or decreases in response to a signal. For example, the c-fos promoter is specifically activated upon binding of growth hormone to its receptor on the cell surface. The tetracycline (tet) promoter containing the tetracycline operator sequence (tetO) can be induced by a tetracycline-regulated transactivator protein (tTA). Binding of the tTA to the tetO is inhibited in the presence of tet (Mosser *et al.* (1997), *supra*). For other inducible promoters including jun, fos and metallothionein and heat shock promoters, see, *e.g.*, Sambrook er *al.*, *supra*; and Gossen *et al.* Inducible gene expression systems for higher eukaryotic cells, in Curr. Opi. Biotech. 5:516-520 (1994). Among the eukaryotic promoters that have been identified as strong promoters for high-level expression arc the SV40 early promoter, adenovirus major late promoter, mouse metallothionein-I promoter, Rous sarcoma virus long terminal repeat, and human cytomegalovirus immediate early promoter (CMV).

An "enhancer", as used herein, refers to a polynucleotide sequence that enhances transcription of a gene or coding sequence to which it is operably linked. Unlike promoters, enhancers are relatively orientation and position independent and have been found 5' (Lainins *et al.,* Proc. Natl. Acad. Sci. USA, 78:993 [1981]) or 3' (Lusky *et al.,* Mol. Cell Bio . 3:1108 [1983]) to the transcription unit, within an intron (Banerji *et al.,* Cell, 33:729 [1983]) as well as within the coding sequence itself (Osborne *et al.,* Mol. Cell Bio., 4:1293 [1984]). Therefore, enhancers may be placed upstream or downstream from the transcription initiation site or at considerable distances from the promoter, although in practice enhancers may overlap physically and functionally with promoters. A large number of enhancers, from a variety of different sources are well known in the art (and identified in databases such as GenBank) and available as or within cloned polynucleotide sequences (from, *e.g.,* depositories such as the ATCC as well as other commercial or individual sources). A number of polynucleotides comprising promoter sequences (such as the commonly-used CMV promoter) also comprise enhancer sequences. For example, all of the strong promoters listed above also contain strong enhancers. Bendig, Genetic Engineering, 7:91 (Academic Press, 1988).

The term "intron" as used herein, refers to a non-coding nucleotide sequence of varying length, normally present within many eukaryotic genes, which is removed from a newly transcribed mRNA precursor by the process of splicing. In general, the process of splicing requires that the 5' and 3' ends of the intron be correctly cleaved and the resulting ends of the mRNA be accurately joined, such that a mature mRNA having the proper reading frame for protein synthesis is produced. An intron useful in the constructs of this invention will generally be an efficient intron characterized by a splicing efficiency which results in most of the transcripts diverted to expression of the desired product while also providing enough unspliced transcripts for expression of the selectable marker gene (selectable marker gene cloned within and bounded by the ends of, the intron) in amounts sufficient for selection. The efficient intron preferably has a splicing efficiency of about 80 to 99%, preferably about 90-99%. Intron splicing efficiency is readily determined by quantifying the spliced transcripts versus the full-length, unspliced transcripts that contain the intron, using methods known in the art such as by quantitative PCR or Northern blot analysis, using appropriate probes for the transcripts. See, *e.g*., Sambrook *et al., supra,* and other general cloning manuals. Reverse transcription-polymerase chain reaction (RT-PCR) can be used to analyze RNA samples containing mixtures of spliced and unspliced mRNA transcripts. For example, fluorescent- tagged primers designed to span the intron are used to amplify both spliced and unspliced targets. The resultant amplification products are then separated by gel electrophoresis and quantitated by measuring the fluorescent emission of the appropriate band(s). A comparison is made to determine the amount of spliced and unspliced transcripts present in the RNA sample.

Introns have highly conserved sequences at or near each end of the intron which are required for splicing and intron removal. As used herein "splice donor site" or "SD" or "5' splice site" refers to the conserved sequence immediately surrounding the exon-intron boundary at the 5' end of the intron, where the exon comprises the nucleic acid 5' to the intron. The term "splice acceptor site" or "SA" or "3' splice site" herein refers to the sequence immediately surrounding the intron-exon boundary at the 3' end of the intron, where the exon comprises the nucleic acid 3' to the intron. An "efficient intron" will comprise a splice donor site and a splice acceptor site that result in splicing of messenger RNA precursors at a frequency between about 80 to 99%, preferably 90 to 95%, more preferably at least 95%, as determined by methods known in the art such as by quantitative PCR. Many splice donor and splice acceptor sites have been charactered and Ohshima *et al*., J. Mol. Biol., 195:247-259 (1987) provides a review of these. Examples of efficient splice donor sequences include the wild type (WT) ras splice donor sequence and the GAC:GTAAGT sequence. One preferred splice donor site is a "consensus splice donor sequence" and a preferred splice acceptor site is a "consensus splice acceptor sequence"; these consensus sequences are evolutionarily highly conserved. The consensus sequences for both splice donor and splice acceptor sites in the mRNAs of higher eukaryotes are shown in Molecular Biology of the Cell, 3^{rd} edition. Alberts *et al*. (eds.), Garland Publishing, Inc., New York, 1994, on page 373, Figure 12-53. The consensus sequence for the 5' splice donor site is C/A (C or A) AG:GUAAGU (wherein the colon denotes the site of cleavage and ligation). The 3' splice acceptor site occurs within the consensus sequence (U/C)_{II}NCAG:G. Other efficient splice donor and acceptor sequences can be readily determined using the techniques for measuring the efficiency of splicing.

An "internal ribosome entry site" or "IRES" describes a sequence which functionally promotes translation initiation independent from the gene 5' of the IRES and allows two cistrons (open reading frames) to be translated from a single transcript in an animal cell. The IRES provides an independent ribosome entry site for translation of the open reading frame immediately downstream (downstream is used interchangeably herein with 3') of it. Unlike bacterial mRNA which can be polycistronic, *i.e*., encode several different polypeptides that are translated sequentially from the mRNAs, most mRNAs of animal cells are monocistronic and code for the synthesis of only one protein. With a polycistronic transcript in a eukaryotic cell, translation would initiate from the 5' most translation initiation site, terminate at the first stop codon, and the transcript would be released from the ribosome, resulting in the translation of only the first encoded polypeptide in the mRNA. In a eukaryotic cell, a polycistronic transcript having an IRES operably linked to the second or subsequent open reading frame in the transcript allows the sequential translation of that downstream open reading frame to produce the two or more polypeptides encoded by the same transcript. The use of IRES elements in vector construction has been previously described, see, *e.g.,* Pelletier *et al.,* Nature 334: 320-325 (1988); Jang *et al.,* J. Virol. 63: 1651-1660 (1989); Davies *et al.,* J. Virol. 66: 1924-1932 (1992); Adam *et al.* J. Virol. 65: 4985-4990 (1991); Morgan *et al.* Nucl. Acids Res. 20: 1293-1299 (1992); Sugimoto *et al.* Biotechnology 12: 694-698 (1994); Ramesh *et al.* Nucl.Acids Res. 24: 2697-2700 (1996); and Mosser *et al.* (1997), *supra*).

"Operably linked" refers to a juxtaposition of two or more components, wherein the components so described are in a relationship permitting them to function in their intended manner. For example, a promoter and/or enhancer is operably linked to a coding sequence if it acts in cis to control or modulate the transcription of the linked sequence. Generally, but not necessarily, the DNA sequences that are "operably linked" are contiguous and, where necessary to join two protein coding regions or in the case of a secretory leader, contiguous and in reading frame. However, although an operably linked promoter is generally located upstream of the coding sequence, it is not necessarily contiguous with it. Enhancers do not have to be contiguous. An enhancer is operably linked to a coding sequence if the enhancer increases transcription of the coding sequence. Operably linked enhancers can be located upstream, within or downstream of coding sequences and at considerable distances from the promoter. A polyadenylation site is operably linked to a coding sequence if it is located at the downstream end of the coding sequence such that transcription proceeds through the coding sequence into the polyadenylation sequence. Linking is accomplished by recombinant methods known in the art, *e.g.*, using PCR methodology, by annealing, or by ligation at convenient restriction sites. If convenient restriction sites do not exist, then synthetic oligonucleotide adaptors or linkers are used in accord with conventional practice.

The term "expression" as used herein refers to transcription or translation occurring within a host cell. The level of expression of a desired product in a host cell may be determined on the basis of either the amount of corresponding mRNA that is present in the cell, or the amount of the desired product encoded by the selected sequence. For example, mRNA transcribed from a selected sequence can be quantitated by PCR or by northern hybridization (see Sambrook *et al.,* Molecular Cloning: A Laboratory Manual, Cold Spring Harbor Laboratory Press (1989)). Protein encoded by a selected sequence can be quantitated by various methods, *e.g.,* by ELISA, by assaying for the biological activity of the protein, or by employing assays that are independent of such activity, such as western blotting or radioimmunoassay, using antibodies that are recognize and bind reacting the protein. See Sambrook *et al.,* 1989, *supra.*

A "host cell" refers to a cell into which a polynucleotide of the invention is introduced. Host cell includes both prokaryotic cells used for propagation of the construct to prepare plasmid stocks, and eukaryotic cells for expression of the selected sequence. Typically, the eukaryotic cells are mammalian cells.

The technique of "polymerase chain reaction," or "PCR," as used herein generally refers to a procedure wherein minute amounts of a specific piece of nucleic acid, RNA and/or DNA, are amplified, as described in U.S. Patent No. 4,683,195 issued 28 July 1987. Generally, sequence information from the ends of the region of interest or beyond needs to be available, such that oligonucleotide primers can be designed; these primers will be identical or similar in sequence to opposite strands on the template to be amplified. Generally, the PCR method involves repeated cycles of primer extension synthesis, using two DNA primers capable of hybridizing preferentially to a template nucleic acid comprising the nucleotide sequence to be amplified. The 5' terminal nucleotides of the two primers may coincide with the ends of the amplified material. PCR can be used to amplify specific RNA sequences, specific DNA sequences from total genomic DNA, and cDNA transcribed from total cellular RNA, bacteriophage or plasmid sequences, etc. See, generally, Mullis *et al.,* Cold Spring Harbor Symp. Quant. Biol., 51:263 (1987); Erlich, ed., PCR Technology, (Stockton Press, NY, 1989); Wang & Mark, pp.70-75 and Scharf, pp. 84-98, both in PCR Protocols, (Academic Press, 1990). As used herein, PCR is considered to be one, but not the only example of a nucleic acid polymerase reaction method for amplifying a nucleic acid test sample, comprising the use of a known nucleic acid (DNA or RNA) as a primer. As used herein, PCR techniques include RT-PCR.

### References

The practice of the present invention will employ, unless otherwise indicated, conventional techniques of molecular biology and the like, which are within the skill of the art. Such techniques are explained fully in the literature. See *e.g*., Molecular Cloning: A Laboratory Manual, (J. Sambrook *et al.,* Cold Spring Harbor Laboratory, Cold Spring Harbor, N.Y., 1989); Current Protocols in Molecular Biology (F. Ausubel *et al.,* eds., 1987 updated); Essential Molecular Biology (T. Brown ed., IRL Press 1991); Gene Expression Technology (Goeddel ed., Academic Press 1991); Methods for Cloning and Analysis of Eukaryotic Genes (A. Bothwell *et al.* eds., Bartiett Publ. 1990); Gene Transfer and Expression (M. Kriegler, Stockton Press 1990); Recombinant DNA Methodology (R. Wu *et al*. eds., Academic Press 1989); PCR: A Practical Approach (M. McPherson *et al.,* IRL Press at Oxford University Press 1991); Oligonucleotide Synthesis (M. Gait ed., 1984); Cell Culture for Biochemists (R. Adams ed., Elsevier Science Publishers 1990); Gene Transfer Vectors for Mammalian Cells (J. Miller & M. Calos eds., 1987); Mammalian Cell Biotechnology (M. Butler ed., 1991); Animal Cell Culture (J. Pollard *et al.* eds., Humana Press 1990); Culture of Animal Cells, 2^{nd} Ed. (R. Freshney *et al.* eds., Alan R. Liss 1987); Flow Cytometry and Sorting (M. Melamed *et al.* eds., Wiley-Liss 1990); the series Methods in Enzymology (Academic Press, Inc.); and Animal Cell Culture (R. Freshney ed., IRL Press 1987); and Wirth M. and Hauser H. (1993) Genetic Engineering of Animal Cells, In: Biotechnology Vol. 2 Puhler A (ed.) VCH, Weinhcim 663-744.

### Modes for Carrying Out the Invention

The invention provides constructs useful for screening, selecting and isolating cells expressing high levels of a gene or sequence of interest. Many variations of the basic construct design are possible and examples will be described in detail below. One of skill in the art will recognize that modifications of the present vectors can be made without departing from the scope of the invention. It will also be understood that desirable features that facilitate cloning can be genetically engineered into the genes of interest and the vectors by methods routine in the art of recombinant DNA methodology.

Amplifiable selectable genes suitable for use in the polynucleotides of the invention are exemplified above, see the section under Definitions. Preferably, the amplifiable selectable gene is the gene encoding DHFR. Transfectants carrying the DHFR gene can be initially selected for and identified by culturing the cells in culture medium that contains Mtx. The transfected cells then are exposed to successively higher amounts of Mtx to select for host cells having undergone amplification resulting in multiple copies of the DHFR gene, and concomitantly, multiple copies of the gene of interest and sequences physically connected to the DHFR sequence (U.S. Patent No. 4,713,339; Axel *et al.,* U.S. Patent No. 4,634.665; Axel *et al.,* U.S. Patent No. 4,399,216; Schimke, J. Biol. Chem., 263:5989 (1988)). DNA encoding DHFR is available; a mouse DHFR cDNA fragment is described in Simonsen and Levinson, Proc. Nat. Acad. Sci. U.S.A. 80:2495-1499 (1983) and in U.S. Patent No. 5,561,053.

Fluorescent proteins and specifically, green fluorescent proteins usable in the invention are described above under Definitions. For a review of GFP, its uses, and microscopy setup and fluorescence filters for detection of GFP fluorescence, see, *e.g.,* Ausubel *et al*. Current Protocols in Molecular Biology, Supplement 34, 1996, Unit 9.7C. A preferred fluorescent protein is GFP, preferably from the jelly fish, *Aequorea victoria*. In one embodiment, the *Aequorea* GFP mutant, S65T, is used. The structure of and cDNA encoding *Aequorea* wild-type GFP is described in Prasher *et al* Gene 111: 229-233 (1992); Chalfie *et al*. (1994), *supra* (This sequence has a change created by PCR; codon 80 changed from Glu to Arg (CAG to CGG). The plasmid pGFP10.1 encoding GFP is available under ATCC accession number 75547 (see Chalfie U.S. Patent No. 5,491,084). For description of nucleic acids encoding mutant GFPs, see, *e.g*., U.S. Patent No. 5,625,048; U.S. Patent No. 5,777,079, U.S. Patent No. 5,804,387, patent publications WO 9806737. WO 9821355, WO 9742320, Chalfie *et al*. WO 9521191. Other green fluorescent protein mutants with increased cellular fluorescence compared to the wild-type protein are described in, *e.g.,* Nataranjan *et al.* J. Biotechnol. 62:29-45 (1998); and Crameri *et al*. Nature Biotechnol. 14:315-319 (1996). Mutant GFPs can be created by random or site-directed mutagenesis of the GFP genes (site-directed mutagenesis can be performed using, *e.g.,* the Muta-Gene phagemid *in vitro* mutagenesis kit from Bio-Rad). Vectors containing various variant GFP genes including GFP linked to CMV promoter are commercially available from, *e*.*g*., Clontech Laboratories, Inc., Palo Alto, CA; and Quantum Biotechnologies Inc., Montreal, Canada. These GFP gene inserts can be excised from the vectors following the manufacturer's instructions.

For a description of the functional components of mammalian expression vectors including specific examples of promoters, enhancers, termination and polyadenylation signals, splicing signals, refer to Sambrook *et al*., 1989, *supra,* Chapter 16: Expression of Cloned Genes in cultured Mammalian Cells.

Each transcription unit will contain a promoter, a transcription termination sequence and a polyA signal sequence downstream of the coding sequences present in that transcription unit. The promoter sequence may overlap with the transcription initiation site. Various polyA sites are known, *e.g*., SV40, Hepatitis B, or BGH (bovine growth hormone) polyA. Additionally, each coding sequence will include its own translational initiation site (AUG) and stop codon. These regulatory elements, if not already present as part of the gene of interest, as well as other desirable features that facilitate cloning, can be genetically engineered into the gene and vectors by methods routine in the art of recombinant DNA methodology.

The construct will contain at least one promoter to drive transcription of the selected sequence encoding the desired product, the amplifiable selectable gene and the green fluorescent protein gene. The promoter used will be one functional in the cell in which expression of the amplifiable selectable gene, green fluorescent protein (GFP) gene and the selected sequence is contemplated. For example, if the host cell is a mammalian cell, the promoter employed will be a promoter functional in mammalian cell, preferably a mammalian or viral promoter. The promoter normally associated with the gene of interest can be used, provided such promoters are compatible with the host cell expression systems.

Viral promoters obtained from the genomes of viruses include promoters from polyoma virus, fowlpox virus (UK 2,211,504 published 5 July 1989), adenovirus (such as Adenovirus 2 or 5), herpes simplex virus (thymidine kinase promoter), bovine papilloma virus, avian sarcoma virus, cytomegalovirus, a retrovirus (*e.g.,* MoMLV, or RSV LTR), Hepatitis-B virus, Myeloproliferative sarcoma virus promoter (MPSV), VISNA, and Simian Virus 40 (SV40). Heterologous mammalian promoters include, *e.g*., the actin promoter, immunoglobulin promoter, heat-shock protein promoters. The aforementioned promoters are known in the art.

The early and late promoters of the SV40 virus are conveniently obtained as a restriction fragment that also contains the SV40 viral origin of replication. Fiers *et al.,* Nature, 273:113 (1978); Mulligan and Berg, Science, 209:1422-1427 (1980); Pavlakis *et al*., Proc. Natl. Acad. Sci. USA, 78:7398-7402 (1981). The immediate early promoter of the human cytomegalovirus (CMV) is conveniently obtained as a *Hind*III E restriction fragment. Greenaway *et al*., Gene, 18:355-360 (1982). A broad host range promoter, such as the SV40 early promoter or the Rous sarcoma virus LTR, is suitable for use in the present expression vectors.

Generally, a strong promoter is employed to provide for high level transcription and expression of the desired product. Among the eukaryotic promoters that have been identified as strong promoters for high-level expression are the SV40 early promoter, adenovirus major late promoter, mouse metallothionein-1 promoter, Rous sarcoma virus long terminal repeat, and human cytomegalovirus immediate early promoter (CMV or CMV IE). In a preferred embodiment, the promoter is a SV40 or a CMV early promoter.

The promoters employed can he constitutive or regulatable, *e.g*., inducible. Exemplary inducible promoters include jun, fos and metallothionein and heat shock promoters. See, *e.g*., Sambrook *et al., supra.* One or both promoters of the transcription units can he an inducible promoter. In one embodiment, the GFP is expressed from a constitutive promoter while an inducible promoter drives transcription of the gene of interest and/or the amplifiable selectable marker.

The transcriptional regulatory region in higher eukaryotes may comprise an enhancer sequence. Many enhancer sequences from mammalian genes are known *e.g.*, from globin, elastase, albumin, α-fetoprotein and insulin genes. A suitable enhancer is an enhancer from a eukaryotic cell virus. Examples include the SV40 enhancer on the late side of the replication origin (bp 100-270), the enhancer of the cytomegalovirus immediate early promoter (Boshart *et al*. Cell 41:521 (1985)), the polyoma enhancer on the late side of the replication origin, and adenovirus enhancers. See also Yaniv, Nature, 297:17-18 (1982) on enhancing elements for activation of eukaryotic promoters. The enhancer sequences may be introduced into the vector at a position 5' or 3' to the gene of interest, but is preferably located at a site 5' to the promoter.

Sometimes, the polynucleotide encoding the selectable gene and/or the gene of interest is preceded by DNA encoding a signal sequence having a specific cleavage site at the N-terminus of the mature protein or polypeptide. In general, the signal sequence may be a component designed into the basic expression vector, or it may be a part of the selectable gene or desired product gene that is inserted into the expression vector. If a heterologous signal sequence is used, it is preferably one that is recognized and processed (*i.e*., cleaved by a signal peptidase) by the host cell. For mammalian cell expression, the native signal sequence of the protein of interest may be used if the protein is of mammalian origin. Alternatively, the native signal sequence can be substituted by other suitable mammalian signal sequences, such as signal sequences from secreted polypeptides of the same or related species, as well as viral secretory leaders, for example, the herpes simplex gD signal. The DNA for such precursor region is operably linked in reading frame to the selectable gene or product gene.

The mammalian expression vectors will typically contain prokaryotic sequences that facilitate the propagation of the vector in bacteria. Therefore, the vector may have other components such as an origin of replication (*i.e*., a nucleic acid sequence that enables the vector to replicate in one or more selected host cells) and antibiotic resistance genes for selection in bacteria. Additions eukaryotic selectable gens(s) may be incorporated. Generally, in cloning vectors the origin of replication is one that enables the vector to replicate independently of the host chromosomal DNA, and includes origins of replication or autonomously replicating sequences. Such sequences are well known, *e*.*g*., the ColE1 origin of replication in bacteria. Various viral origins (SV40, polyoma, adenovirus, VSV or BPV) are useful for cloning vectors in mammalian cells. Generally, a eukaryotic replicon is not needed for expression in mammalian cells unless extrachromosomal (episomal) replication is intended (the SV40 origin may typically be used only because it contains the early promoter).

The present constructs can accommodate a wide variety of nucleotide sequence inserts. To facilitate insertion and expression of different genes of interest from the constructs and expression vectors of the invention, the constructs are designed with at least one cloning site for insertion of any gene of interest. Preferably, the cloning site is a multiple cloning site, *i.e.*, containing multiple restriction sites. DNA cassettes containing multiple cloning sites can be isolated from commercially available cloning vectors.

Each construct or expression vector will contain at least one selected sequence encoding a product of interest. In a specific embodiment, the expression vector will contain two selected sequences in separate transcription units, for expressing two desired products, *e.g.,* a heavy and a light chain of an immunoglobulin.

The "selected sequence" encodes a desired product such as a protein, polypeptide, peptide, or a fragment thereof, or even an antisense RNA. The polypeptide can be a subunit of a multichain protein, *e.g.*, an immunoglobulin or a receptor. In a preferred embodiment, the desired product is of human origin or humanized, such as humanized antibodies, and chimeric or fusion proteins having human portions. The chimeric or fusion proteins include Ig-fusion proteins and proteins fused to a tag or other label such as a polyhistidine tag or an epitope tag. Various tags are known in the art. In one embodiment, the desired product is a therapeutic protein or peptide. In a preferred embodiment, the protein is a secreted protein. Secreted or soluble forms of normally membrane bound proteins can be produced from truncated genes in which the sequences encoding the transmembrane domain have been deleted. For example, the secreted polypeptide can comprise the extracellular domain(s) (ECD) of the full length genes.

Examples of mammalian polypeptides or proteins include hormones, cytokines and lymphokines, antibodies, receptors, adhesion molecules, and enzymes. A non-exhaustive list of desired products include, *e*.*g*., human growth hormone, bovine growth hormone, parathyroid hormone, thyroid stimulating hormone, follicle stimulating hormone growth, luteinizing hormone; hormone releasing factor; lipoproteins; alpha-1-antitrypsin; insulin A-chain; insulin B-chain; proinsulin; calcitonin; glucagon; molecules such as renin; clotting factors such as factor VIIIC, factor IX, tissue factor, and von Willebrands factor; anti-clotting factors such as Protein C, atrial natriuretic factor, lung surfactant; a plasminogen activator, such as urokinase or human urine or tissue-type plasminogen activator (t-PA); bombesin; thrombin; hemopoietic growth factor; tumor necrosis factor-alpha and -beta; enkephalinase; RANTES (regulated on activation normally T-cell expressed and secreted); human macrophage inflammatory protein (MIP-1-alpha); a serum albumin such as human serum albumin; mullerian-inhihiting substance; relaxin A- or B-chain; prorelaxin; mouse gonadotropin-associated peptide; DNase; inhibin; activin; receptors for hormones or growth factors; integrin; protein A or D; rheumatoid factors; a neurotrophic factor such as hone-derived neurotrophic factor (BDNF), neurotrophin-3, -4, -5, or -6 (NT-3, NT-4, NT-5, or NT-6), growth factors including vascular endothelial growth factor (VEGF), nerve growth factor such as NGF-β; platelet-derived growth factor (PDGF); fibroblast growth factor such as aFGF, bFGF, FGF-4, FGF-5, FGF-6; epidermal growth factor (EGF); transforming growth factor (TGF) such as TGF-alpha and TGF-beta, including TGF-β1, TGF-β2, TGF-β3, TGF-β4, or TGF-β5; insulin-like growth factor-I and -II (IGF-I and IGF-II); des(1-3)-IGF-I (brain IGF-I), insulin-like growth factor binding proteins: CD proteins such as CD-3, CD-4, CD-8, and CD-19; erythropoietin; osteoinductive factors; immunutoxins; a bone morphogenetic protein (BMP); an interferon such as interferon-alpha, -beta, and -gamma; colony stimulating factors (CSFs), *e*.*g*.*,* M-CSF, GM-CSF, and G-CSF; interleukins (ILs), *e.g.,* IL-1 to IL-10; superoxide dismutase; T-cell receptors; surface membrane proteins *e.g*., HER2; decay accelerating factor; viral antigen such as, for example, a portion of the AIDS envelope; transport proteins; homing receptors; addressins: regulatory proteins; antibodies; chimeric proteins such as immunoadhesins and fragments of any of the above-listed polypeptides. Examples of bacterial polypeptides or proteins include, *e.g*., alkaline phosphatase and β-lactamase.

Preferred polypeptides and proteins herein are therapeutic proteins such as TGF-β, TGF-α, PDGF, EGF, FGF, IGF-I, DNase, plasminogen activators such as t-PA, clotting factors such as tissue factor and factor VIII, hormones such as relaxin and insulin, cytokines such as IFN-γ, chimeric proteins such as TNF receptor IgG immunoadhesin (TNFr-IgG) or antibodies such as anti-IgE. Preferred therapeutic proteins are those of human origin or "humanized" proteins such as humanized antibodies. In specific embodiments, the selected sequence encodes a protein selected from the group consisting of neuronotrophin-3, deoxyribonuclease, vascular endothetial growth factor, HER2 receptor, and immunoglobulin.

Desired product genes or sequences may be obtained from phage display libraries, cDNA or genomic DNA libraries. The gene or sequence of interest can be isolated by PCR methods using suitable primers, or they can be chemically synthesized. Libraries can be screened with probes (such as antibodies or oligonucleotides) designed to identify the selectable gene or the product gene (or the protein(s) encoded thereby). Screening the cDNA or genomic library with the selected probe may be conducted using standard procedures as described in chapters 10-12 of Sambrook *et al*., Molecular Cloning: A Laboratory Manual (New York: Cold Spring Harbor Laboratory Press, 1989).

It is understood that the elements described above are linked in proper reading frame. Further, it is understood that the vectors of the invention can have addition of sequences and sites that facilitate construction and cloning or optimize expression in the selected host cell.

Most expression vectors are "shuttle" vectors, *i*.*e*., they are capable of replication in at least one class of organism but can be transfected into another organism for expression. For example, a vector is cloned in *E. coli* and then the same vector is transfected into yeast or mammalian cells for expression even though it is not capable of replicating independently of the host cell chromosome.

For analysis to confirm correct sequences in the constructs, plasmids from the transformants are prepared, analyzed by restriction, and/or sequenced by methods known in the art.

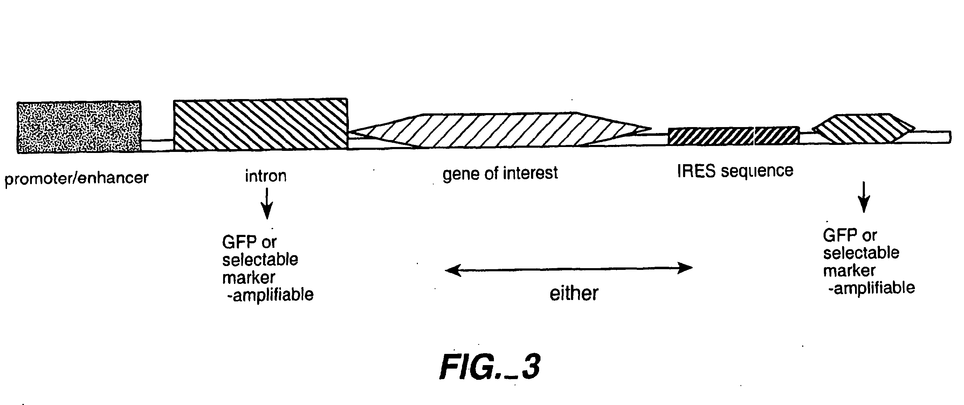

Figures 1 through 6 show schematically, examples of he various configurations of the elements in expression vectors some of which are embodiments of the invention. The configuration of e GFP and amplifiable selectable marker (and any additional selectable marker) as well as the nature of the promoter/enhancer regions that are optimal for expression of a particular desired protein can be readily determined by one of skill in the art by testing various configurations and elements and comparing the resultant productivity of the desired protein. For convenience, the examples that follow will refer to the DHFR gene and gene fusions but it will be understood that any suitable amplifiable selectable gene can substitute for DHFR. Whether the construct has one or more transcription units, each of the transcription units will comprise the elements necessary for the transcription and translation in the appropriate host cells, of the selected sequence, GFP and amplifiable selectable marker genes within that unit. These elements, if not already present as part of the gene, can be genetically engineered into the constructs by methods well known in the art of recombinant DNA methodology. Generally, the promoter and other transcriptional and translational regulatory elements will be selected to optimize the level of expression and secretion (where relevant), of the desired product. The regulatory elements in the second transcription unit can be the same as those used in the first transcription unit, *e.g*., the SV40 promoter and the same source of polyA signal sequence can be cloned into both the first and second transcription units.

A polynucleotide comprising a single transcription unit from which the amplifiable selectable marker, the desired protein, and GFP are expressed, is exemplified in Figure 1, rows land 2. In the construct with the single transcription unit, the promoter and optionally, an enhancer, are placed upstream from sequences coding for a desired protein, an amplifiable selectable marker, and GFP. The enhancer is conveniently, but does not have to be placed contiguous with the promoter to be active in enhancing transcription. A transcription termination sequence and polyA signal are present downstream of the three components (the amplifiable selectable marker, selected sequence and GFP genes). The sequence containing the polyA signal present in the constructs described in the working examples below, includes a transcription termination site.

DHFR, the desired protein and GFP can be expressed from one promoter to improve the co-expression efficiency. For example, GFP and DHFR can be expressed as a fusion protein, or an IRES can obviate the need for a second promoter to express GFP. In the constructs shown in Figure 1, rows 1 and 2, the exemplary amplifiable selectable gene, DHFR, is fused to the GFP gene to form a DHFR-GFP fusion gene. Each of the upstream and downstream coding sequences (in the first example in Figure 1, row 1, the upstream coding sequence is DHFR-GFP fusion gene; in the second example represented in row 2, the upstream coding sequence is the selected sequence) has its translational stop signal. Translation initiates again for the downstream coding sequence. These scenarios allow expression of two separate proteins from a single promoter. It will be understood that the positioning of the promoter/enhancer, translational stop signal, translational initiation site, transcription termination site and polyA signal, relative to the various components in each transcription unit, as described here, apply to all the constructs described below.

The polynucleotides of the invention will preferably be configured to divert most of the transcript to expression of the desired product while linking it, at a fixed ratio, to expression of the amplifiable selectable gene to allow selection of stable transfectants. For mammalian expression vectors, it is preferred to have an intron 5' of a gene (gene of interest, GFP or other selectable gene) for improved expression. Intron-modified selectable genes comprising the coding sequence of a selectable gene and an intron that reduces the level of selectable protein produced from the selectable gene. (WO 92/17566; Abrams *et al.* J. Biol. Chem. 264(24):14016-14021 (1989).

Preferably, the intron present in the constructs of the invention has efficient splice donor and acceptor sites, as defined above, such that splicing of the primary transcript occurs at a frequency greater than 90%, preferably at least 95%. In this manner, at least 95% of the transcripts will be translated into desired product, and 5% or less into the amplifiable selectable marker if one is placed in the intron. In one embodiment, an intron having consensus splice donor and acceptor sites is used. The introns suitable for use in the present constructs will generally be at least 91 nucleotides long, preferably at least about 150 nucleotides, since introns which are shorter than this tend to be spliced less efficiently. The upper limit for the length of the intron can be up to 30 kb or more. However, the intron used in herein is generally less than about 10 kb in length.

Introns suitable for use in the present invention are suitably prepared by any of several methods that are well known in the art, such as isolation from a naturally occurring nucleic acid or *de novo* synthesis. The introns present in many naturally occurring eukaryotic genes have been identified and characterized. Mount, Nucl. Acids Res., 10:459 (1982). Artificial introns comprising functional splice sites also have been described. Winey *et al*., Mol. Cell Biol., 9:329 (1989); Gatermann *et al*., Mol. Cell Biol., 9:1526 (1989). Introns may be obtained from naturally occurring nucleic acids, for example, by digestion of a naturally occurring nucleic acid with a suitable restriction endonuclease, or by PCR cloning using primers complementary to sequences at the 5' and 3' ends of the intron. Alternatively, introns of defined sequence and length may be prepared by *in vitro* deletion mutagenesis of an existing intron, or synthetically using various methods in organic chemistry. Narang *et al*., Meth. Enzymol., 68:90 (1979); Caruthers *et al*., Meth. Enzymol., 154:287 (1985); Froehler *et al*., Nucl. Acids Res., 14:5399 (1986).

In one embodiment, the intron used is the intron of the vector pRK which contains a SD derived from the CMV immediate early gene and a SA site from an IgG H chain variable region gene, as described in Lucas *et al*., Nucl. Acids Res. 24: 1774-1779 (1996), Suva *et al*., Science 237: 893-896 (1997), and U.S. Patent No. 5,561,053. The selectable gene or fusion gene is inserted within the intron using any of the various known methods for modifying a nucleic acid *in vitro.* Genes can be inserted into the intron outside of the consensus sequence and without interrupting the sequences important for splicing. Typically, a selectable gene will be introduced into an intron by first cleaving the intron with a restriction endonuclease, and then covalently joining the resulting restriction fragments to the selectable gene in the correct orientation for host cell expression, for example by ligation with ligase. If convenient restriction sites are lacking within the intron, they can be introduced using linkers and oligonucleotides by PCR, ligation or restriction and annealing. An example of intron modification is described in Lucas *et al*., 1996, *supra.*

The IRES can be of varying length and from various sources, *e.g.,* encephalomyocarditis virus (EMCV) or picornavirus genomes. Various IRES sequences and their construction are described in, *e.g.,* Pelletier *et al*., Nature 334: 320-325 (1988); Jang *et al*., J. Virol. 63: 1651-1660 (1989); Davies *et al*., *J.* Virol. 66: 1924-1932 (1992); Adam *et al*. J. Virol. 65: 4985-4990 (1991); Morgan *et al*. Nucl. Acids Res. 20: 1293-1299 (1992); Sugimoto *et al*. Biotechnology 12: 694-698 (1994); and Ramesh *et al.* Nucl. Acids Res. 24: 2697-2700 (1996); and Mosser *et al.* (1997), *supra*). In one embodiment, the IRES of ECMV is used in the vectors of the invention. The downstream coding sequence will be operably linked to the IRES, for example, at about 8 bases or more downstream of the 3' end of the IRES or at any distance that will not negatively affect the expression of the downstream gene. The optimum or permissible distance between the IRES and the start of the downstream gene can be readily determined by varying the distance and measuring expression as a function of the distance.

Instead of fusing the amplifiable selectable gene with the GFP gene, the two genes can be present separately in the single transcription unit. Thus, a third construct design, illustrated in Figure 1, row 3, will comprise in order from 5' to 3', an intron followed by a selected sequence, and an IRES. In one embodiment, the DHFR gene is positioned within the intron, and the GFP gene is placed downstream of the IRES. In such a construct, the primary, unspliced transcript will encode all three components but only the DHFR and the GFP genes will be translated. However, the DHFR sequences will be spliced out of the primary transcript at a high frequency and the resultant spliced transcript will be translated to produce the desired product and GFP. In an alternative embodiment, the GFP gene is placed within the intron and the DHFR gene is downstream of the IRES.

The constructs can comprise two expression/transcription units, as shown in Figure 1, rows 4-9. The two-transcription unit construct depicted in Figure 1, row 4, comprises one selected sequence. Rows 5-9 show constructs wherein two selected sequences can be inserted, one in each transcription unit. Each of the two transcription units will comprise a promoter and optionally, an enhancer, a transcriptional termination site and polyA signal sequence. The second transcription unit can use the same or different kind of promoter as used in first transcription unit. For example, both transcription units can use the SV40 promoter. One or both of the transcription units can comprise an intron.

Figure 1, row 4, illustrates a construct wherein the first transcription unit contains DHFR in an intron (the first intron), followed by the selected sequence. The second transcription unit will comprise the GFP gene. The second transcription unit will preferably comprise an intron (referred to as the second intron) immediately 5' of the GFP. The three coding sequences are still physically linked in one vector but are independently transcribed from two promoters. The primary transcript produced from the first transcription unit encodes both DHFR and the selected sequence but only the DHFR gene is translated into product. Preferably, at least 95% of the transcripts will have the DHFR gene spliced out and will translate into the desired product. In the second transcription unit, if the GFP is placed downstream of an intron, both spliced and unspliced transcripts from this transcription unit will produce GFP.

Where the DHFR and GFP genes are expressed from separate transcription units, their positions are interchangeable so that DHFR gene can be placed in the first transcription unit and GFP, in the second transcription unit, or vice versa.

The preceding construct comprising two transcription units, each with an intron, is useful for expression of two genes of interest, as depicted in Figure 1, row 5. The second transcription unit can comprise a second selected sequence, and the GFP gene in the second intron, both coding sequences operably linked to and transcribed from the same promoter.

In yet another embodiment of the preceding construct comprising two transcription units and two introns, instead of placing the GFP gene within the second intron in the second transcription unit, an IRES is placed between the second selected sequence and the GFP gene (Fig. 1, row 6). Both the second selected sequence and the GFP gene from the second transcription unit will be translated from the dicistronic message.

In yet another alternative configuration of the preceding construct comprising two transcription units and two introns, a DHFR-GFP fusion gene is placed within the first intron (Fig. 1, row 7). The second intron can be without any insert (indicated as empty in the figures) or another selectable marker gene can be inserted within the intron.

In still another variation of the construct comprising two-transcription units and two introns, the first intron in the first transcription unit is left empty but an IRES is inserted downstream of the first gene of interest to allow translation of a downstream DHFR-GFP fusion gene. The second transcription unit will comprise the second intron followed by a second gene of interest (Fig. 1, row 8). Optionally, another selectable marker gene (other than the amplifiable selectable gene and GFP gene), can be placed within the second intron or the intron can remain without an inserted gene.

Finally, the first transcription unit can comprise in order of 5' to 3', a first intron, the first selected sequence, an IRES and DHFR; the second transcription unit can comprise a second intron, a second selected sequence, an IRES and the GFP gene in that order (Fig. 1, row 9).

Expression vectors with two or more transcription units are useful for expression of proteins that are heterodimeric or multichain. The first and second selected sequences in the vector can encode the two polypeptide chains of a heterodimeric receptor. For example, the first selected sequence in the first transcription unit can encode an immunoglobulin heavy (H) chain and the second selected sequence in the second transcription unit encodes the immunoglobulin light (L) chain. For expression of antibody H and L chain, the a preferred configuration is the placement of the selectable marker (DHFR or puromycin-DHFR fusion) in the intron 5' to the H chain and the GFP gene in the intron 5' of the L chain.

### Transfection and Host Cells

The plasmids can be propagated in bacterial host cells to prepare DNA stocks for subcloning steps or for introduction into eukaryotic host cells. Transfection of eukaryotic host cells can be any performed by any method well known in the art and described, *e.g.,* in Sambrook *et al., supra*. Transfection methods include lipofection, electroporation, calcium phosphate co-precipitation, rubidium chloride or polycation (such as DEAE-dextran) -mediated transfection, protoplast fusion and microinjection. Preferably, the transfection is a stable transfection. The transfection method that provides optimal transfection frequency and expression of the construct in the particular host cell line and type, is favored. Suitable methods can be determined by routine procedures. For stable transfectants, the constructs are integrated so as to be stably maintained within the host chromosome.

Host cells suitable for expression of the selected sequence and the amplifiable selectable marker include eukaryotic cells, preferably mammalian cells. Insect and plant cells can also be used with appropriate promoters (*e.g*., baculovirus promoter in Sf9 insect cells). The cell type should be capable of expressing the construct encoding the desired protein, processing the protein and transporting a secreted protein to the cell surface for secretion. Processing includes co- and post-translational modification such as leader peptide cleavage, GPI attachment, glycosylation, ubiquitination, and disulfide bond formation. Immortalized host cell cultures amenable to transfection and *in vitro* cell culture and of the kind typically employed in genetic engineering are preferred. Examples of useful mammalian host cell lines are monkey kidney CV1 line transformed by SV40 (COS-7, ATCC CRL 1651); human embryonic kidney line (293 or 293 derivatives adapted for growth in suspension culture, Graham *et al*., J. Gen Virol., 36:59 (1977); baby hamster kidney cells (BHK, ATCC CCL 10); DHFR⁻Chinese hamster ovary cells (ATCC CRL-9096) ; dp12.CHO cells, a derivative of CHO/DHFR. (EP 307,247 published 15 March 1989); mouse sertoli cells (TM4, Mather, Biol. Reprod., 23:243-251 (1980)); monkey kidney cells (CVI ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather *et al*., Annals N.Y. Acad. Sci., 383:44-68 (1982)); PEER human acute lymphoblastic cell line (Ravid *et al*. Int. J. Cancer 25:705-710 (1980)); MRC 5 cells; FS4 cells; human hepatoma line (Hep G2), human HT1080 cells, KB cells, JW-2 cells, Detroit 6 cells, NIH-3T3 cells, hybridoma and myeloma cells. Embryonic cells used for generating transgenic animals are also suitable (*e.g*., zygotes and embryonic stem cells).

A suitable host cell when a wild-type DHFR gene is used is the Chinese Hamster Ovary (CHO) cell line deficient in DHFR activity, ATCC CRL-9096, prepared and propagated as described by Urlaub & Chasin, Proc. Nat. Acad. Sci. USA, 77:4216 (1980), as well as derivatives of this cell line including the dp12 cell line. To extend the DHFR amplification method to other cell types, a mutant DHFR gene that encodes a protein with reduced sensitivity to methotrexate may be used in conjunction with host cells that contain normal numbers of an endogenous wild-type DHFR gene (see, Simonsen and Levinson, Proc. Natl. Acad. Sci. USA, 80:2495 (1983); Wigler *et al.,* Proc. Natl. Acad. Sci. USA, 77:3567-3570 (1980); Haber and Schimke, Somatic Cell Genetics, 8:499-508 (1982)).

### Screening and selection

Bacteria transformed with the GFP gene can be screened for fluorescence using a long-wave UV lamp.

After transfection of mammalian cells, the cells will typically be grown for about 2 days in nonselective medium. The cells are placed in selection medium about 18-48 hours post-transfection and maintained in selective culture for about 2-4 weeks. If a second selectable marker gene other than the amplifiable selectable gene is present in the expression vector, the cells can be selected for expression of both marker genes simultaneously by adding both selective agents to the culture medium. For example, cells can be selected for DHFR expression in the presence of methotrexate, and concurrently for hygromycin resistance. The culture conditions, such as temperature, pH, and the like, are those previously used with the host cell selected for expression, and will be apparent to the ordinarily skilled artisan. Cells that survive selection are then screened for fluorescence, *e.g.,* by FACS.

The selection of recombinant host cells that express high levels of a desired protein generally is a multi-step process. Transfected cells are screened for expression of the GFP and/or the amplifiable selectable marker to identify cells that have incorporated the expression vector. Typically, the transfected host cells are subjected to selection for expression of the selectable marker(s) by culturing in selection medium for about 2 weeks. Following that, the surviving cells are pooled for screening and sorting by flow cytometry or fluorescence microscopy for expression of GFP. The flow cytometers will generally be fitted with fluorescein isothiocyanate (FITC) filters to detect fluorescence. The cells arc typically subjected to several rounds of sequential sorts, preferably at least two rounds. The brightest cells from the early FACS sorts can be pooled for subsequent culturing and further sorting; however, in the final sort, individual clones are separated out. Repeated sorting enriches the high, stable fluorescence cell population. Typically cells are grown for about 1-3 weeks, more typically 2 weeks in between sorts, depending on the rate of growth of the particular host cell.. Any number or percentage of fluorescent cells can be sorted. Typically, the brightest 1-10% of fluorescent cells (fluorescence intensity measured in units mfe as determined by FACS analysis) within the population analyzed are sorted out at the first sort and second sorts, with fewer numbers of cells sorted out in subsequent sorting steps. For example, in the first sort, the brightest 5% of fluorescent cells are sorted, in the second sort, the brightest 1% of cells are collected and in the third sort, the top 0.5% of cells are isolated are cloned. Suspension or adherent cells are typically sorted in phosphate buffered saline (PBS) and collected in growth medium. The sorted cells can be cultured with or without selection. Fluorescence sorting and selection/amplification can be performed sequentially or simultaneously

Fluorescence microscopy to detect fluorescence is taught in the art, see, *e.g*., Bennett *et al.,* Biotechniques 24: 478-482 (1998). Flow cytometry method for detection of fluorescent cells and analysis of GFP can be performed as described in the examples below, or in the literature, see, *e.g*., Subramanian and Srienc, 1996, *supra,* Ropp *et al.,* Cytometry 21: 309-317 (1995); Nataranjan *et al.* J. Biotechnol. 62: 29-45 (1998); Mosser *et al.* p. 152 (1997), *supra.* Briefly, the transfected cells are illuminated at a wavelength of light appropriate for the particular GFP mutant protein, under conditions such that the GFP emits visible fluorescent light. The excitation and emission wavelength will vary with the particular fluorescent protein used and will generally be described by the manufacturer/supplier of the GFP mutant. Fluorescence intensity is measured using, *e*.*g*., a FACSCAN or a FACSCalibur flow cytometer.

After fluorescence sorting, individual clones are cultured in appropriate selection medium to select for clones that have undergone amplification of at least the amplifiable selectable gene, and usually neighboring sequences physically linked to it as well. The concentration of both selection drug and cells suitable for selection of "amplified" cells will vary with the cell line and can be determined by routine methods, such as by varying the drug concentration or the number of cells to obtain generally about 5% survival in a drug killing curve. It is preferable to keep a low drug concentration while varying the cell number.

The selection agent used in conjunction with a DHFR gene is methotrexate (Mtx) and brightly fluorescent cells are selected for amplification of the DHFR gene and the product gene by exposure to successively increasing amounts of Mtx. Transfected cells are cultured in GHT free medium containing Mtx at an initial concentration typically in the range of between about 1nM to 1000 nM, more typically between 50 nM to 500 nM. The concentration of Mtx can be increased gradually by increments of *e.g*., 100 nM. Less than 100% survival or confluency should be obtained.

Transfectants that survive the drug selection and preferably, also show high fluorescence, can then be analyzed to confirm synthesis of the desired product by analyzing the proteins or mRNA.

### Analysis of transfectants

Gene amplification and/or expression may be measured in a sample directly, for example, by conventional Southern blotting, Northern blotting to quantitate the transcription of mRNA (Thomas, Proc. Natl. Acad. Sci. USA, 77:5201-5205 [1980]), dot blotting (DNA analysis), or *in situ* hybridization, using an appropriately labeled probe, based on the sequences provided herein. Various labels may be employed, most commonly radioisotopes, particularly ³²P. However, other techniques may also be employed, such as using biotin-modified nucleotides for introduction into a polynucleotide. The biotin then serves as the site for binding to avidin or antibodies, which may be labeled with a wide variety of labels, such as radionuclides, fluorescens, enzymes, or the like. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Protein titer can be assayed by various methods known in the art, *e.g.,* by Elisa using *e.g.,* an antibody, ligand, receptor or any binding partner of the desired protein. Presence of the desired product can also be assayed by a functional assay. For example, if the desired product is a secreted enzyme, the functional assay would comprise assaying the cell supernatant for enzymatic action on a substrate. Other immunological methods, such as immunoprecipitation, Western blotting and probing with antibody, immunohistochemical staining of tissue sections and assay of cell culture or body fluids, can be used to quantitate directly the expression of gene product. With immunohistochemical staining techniques, a cell sample is prepared, typically by dehydration and fixation, followed by reaction with labeled antibodies specific for the gene product coupled, where the labels are usually visually detectable, such as enzymatic labels, fluorescent labels, luminescent labels, and the like. A particularly sensitive staining technique suitable for use in the present invention is described by Hsu *et al*., Am. J. Clin. Path., 75:734-738 (1980). The proteins present in the supernatant or lysate can be labeled directly or indirectly. Biosynthetic and other methods of labeling proteins are known in the art.

Transcription levels arc useful indirect indicators of the level of desired protein synthesis. RNA can be analyzed by routine procedures such as PCR, RT-PCR, or Northern blot analysis, using appropriate primers, oligonucleotides or probes. In the preferred embodiment, the mRNA is analyzed by quantitative PCR which is useful to determine the efficiency of splicing, and protein expression is measured using ELISA.

The protein of interest is preferably recovered from the culture medium as a secreted polypeptide, or it can be recovered from host cell lysates if expressed without a secretory signal. When the product gene is expressed in a recombinant cell other than one of human origin, the product of interest is completely free of proteins or polypeptides of human origin. However, it is necessary to purify the product of interest from recombinant cell proteins or polypeptides to obtain preparations that are substantially homogeneous as to the product of interest. As a first step, the culture medium or lysate is centrifuged to remove particulate cell debris. The product of interest thereafter is purified from contaminant soluble proteins and polypeptides, for example, by fractionation on immunoaffinity or ion-exchange columns; ethanol precipitation; reverse phase HPLC; chromatography on silica or on a cation exchange resin such as DEAE; chromatofocusing; SDS-PAGE; ammonium sulfate precipitation; gel electrophoresis using, for example, Sephadex G-75; chromatography on plasminogen columns to bind the product of interest and protein A Sepharose columns to remove contaminants such as IgG.

A kit containing one or more polynucleotides of the invention in a suitable vessel such as a vial may be provided. The polynucleotides including expression vectors, can contain at least one cloning site for insertion of a selected sequence of interest, or can have a specific gene of interest already present in the vector. In one embodiment, the polynucleotide in the kit contains two transcription units with the DHFR gene in the intron of one transcription unit and the GFP gene downstream of the second intron in a second transcription unit. The polynucleotide can be provided in a dehydrated or lyophilized form, or in an aqueous solution. The kit can include a buffer for reconstituting the dehydrated polynucleotide. Other reagents can be included in the kit, *e.g*., reaction buffers, positive and negative control vectors for comparison. Generally, the kit will also include instructions for use of the reagents therein.

The invention will be more fully understood by reference to the following examples, which are intended to illustrate the invention.

### EXAMPLES

### Abbreviations

CHO, Chinese hamster ovary; dNTP, deoxyribonucleoside triphosphate; DHFR, dihydrofolate reductase; DNase, deoxyribonuclease; ELISA, enzyme-linked immunosorbant assay; FACS, fluorescence-activated cell sorter; FAM, 6-carboxyfluorescein; FBS, fetal bovine serum; GFP, green fluorescent protein; GHT, glycine, hypoxanthine and thymidine; IRES, internal ribosomal entry site; kb, kilobase; kDa, kilodalton; mfe, million fluorescein equivalence; MTX, methotrexate; NT3, neuronotrophin-3; PBS, phosphate buffered saline; PCR, polymerase chain reaction; RNase, ribonuclease; RT-PCR, reverse transcriptase polymerase chain reaction; TAMRA, 6-carboxy-tetramethyl-rhodamine; VEGF, vascular endothelial growth factor.

### Example 1

Example I describes the construction and expression of various desired proteins, green fluorescent protein (GFP), and DHFR, from a single vector in Chinese hamster ovary (CHO) cells. The experiments demonstrated that high producing clones could be obtained by FACS sorting based on GFP expression. A two promoter system was used to express the desired protein and GFP. DHFR and the desired protein were expressed from one transcription unit, and GFP from a separate transcription unit (Fig. 1 and Fig. 6).

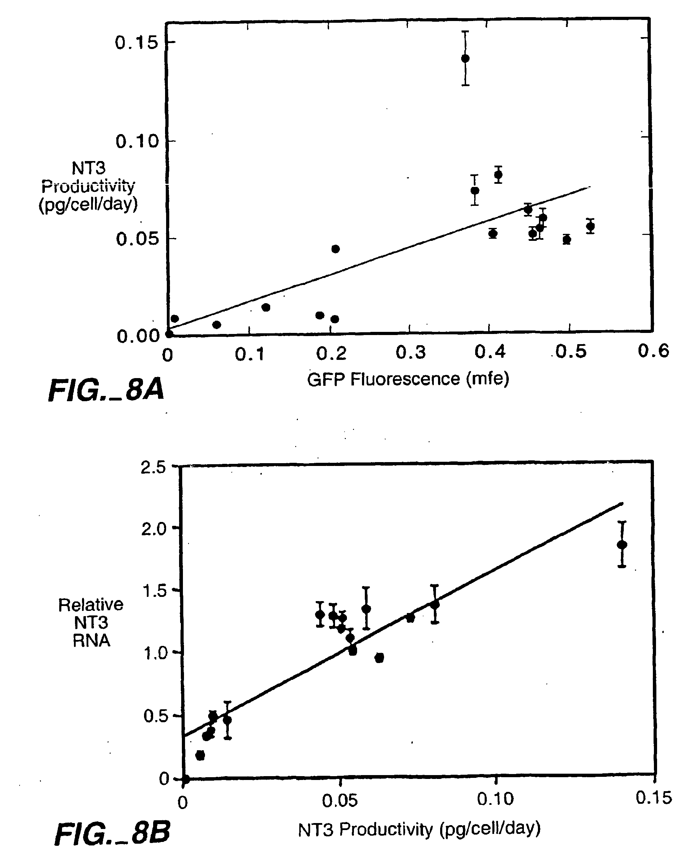

Transfected cells were grown in selection medium and sorted for fluorescence of GFP and cloned by FACS. The following different, desired proteins (enzyme and growth factors) were expressed from this representative expression vector: neuronotrophin-3 (NT3), deoxyribonuclease (DNase), and vascular endothelial growth factor (VEGF). FACS sorting greatly increased the chance of obtaining high producing clones. Overall, a good correlation between the desired protein RNA and GFP RNA and between productivity of the desired protein and GFP fluorescence were seen in the desired protein-GFP producing clones (see Figs. 8A-B, 9A-B, and 11A-D), demonstrating a good co-expression efficiency of two linked transcription units.

### 1. Materials and Methods

### 1.1 Construction of plasmids

As described in Lucas *et al.,* in Nucleic Acid Res. 24: 1774-1779 (1996), a vector containing the DHFR gene in the intron was constructed by inserting the mouse DHFR cDNA into the intron of the expression vector, pRK (Suva *et al.,* Science 237: 893-896 (1987)). Expression vector pRK is driven by the CMV immediate early gene promoter and enhancer (CMV IE P/E) and has a splice donor site from the CMV IE gene and a splice acceptor site from an IgG heavy chain variable region gene (Eaton *et al.* Biochem 25: 8343-8347 (1986). An EcoRV site was inserted into a BstX1 site present 36 bases downstream of the SD of the 144 bp intron of pRK. A 678 bp blunt ended fragment that contained the mouse DHFR cDNA (Simonsen and Levinson (1983), *supra*) was inserted into the EcoRV site.

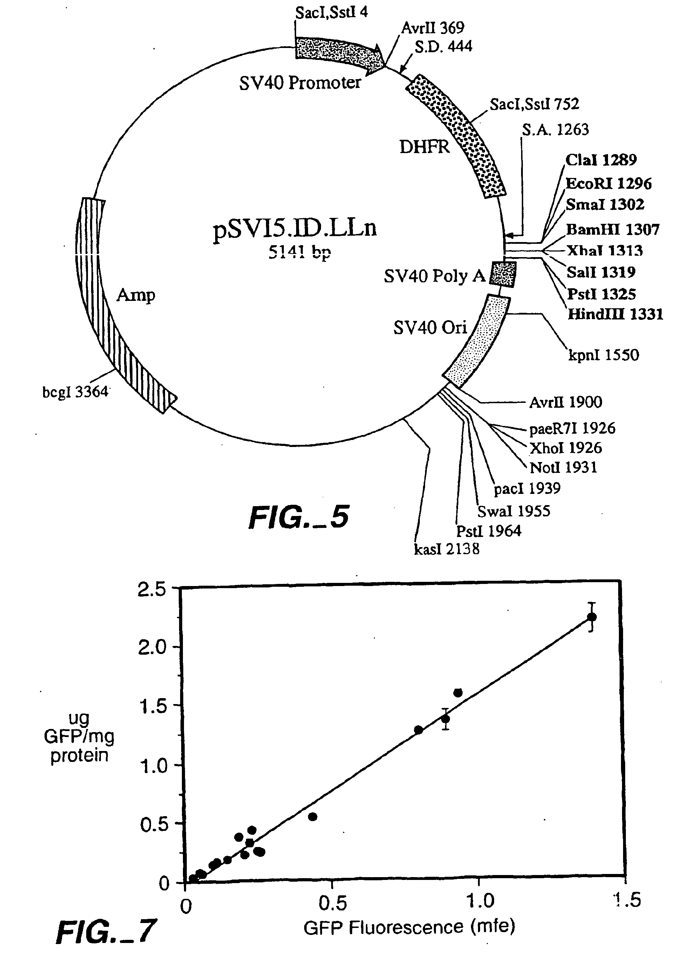

Figure 5 shows a DHFR intron vector, pSV15.ID.LLn (Lucas *et al.,* (1996), *supra*) which is 5141 bp in size and contains a cloning linker region (ClaI through HindIII multiple cloning site) indicated in bold. The vector pSV17.ID.LLn is identical to this vector except that the multiple cloning site is inverted so that the HindIII site is at position 1289 and ClaI at 1331 (not shown).

To express GFP with DHFR alone, an *EcoR*I*-Hind*III fragment from pCMV.S65T.GFP (Ropp *et al*., Cytometry 21 : 309-317 (1995)) containing cDNA encoding GFPS65T was inserted into a cloning linker region of the dicistronic DHFR intron vector described in Lucas *et al*., (1996), *supra.*

To express a desired protein (e.g., NT3, DNase or VEGF) with GFP, the *Avr*II 1900 site downstream from the cloning linker region of the DHFR intron vector was converted to a *Spe*I site. This modified vector was digested with *Avr*II at 369 and *Kpn*I at 1550 and the 4 kb *Kpn*I*-Avr*II backbone fragment was isolated. Previously, NT3, DNase or VEGF₁₆₅ cDNA was cloned into the DHFR intron vector. A 2 kb *Avr*II*-Kpn*I fragment containing cDNA encoding DHFR and one of NT3, DNase or VEGF was isolated from these vectors and ligated with the *Kpn*I*-Avr*II backbone fragment mentioned above to obtain NT3, DNase or VEGF expression vectors with a unique *Spe*I site. From a vector similar to that in pSV15.ID.LLn except without the DHFR gene, an AvrII-AvrII fragment containing the cDNA encoding GFPS65T and the SV40 polyA was cloned into the *Spe*I site to obtain a second transcription unit to express GFP under the second SV40 promoter present 5' of the GFP in the vector. Figure 6 shows an example of the two transcription unit vector for expressing VEGF. Each of DHFR, gene of interest, and GFP has its ATG initiation site.

### 1.2. Cell culture and transfections

DP12 cells, a CHO K1 DUX B11 (DHFR-) derivative, were grown in 50:50 F12/DMEM medium supplemented with 2 mM L-glutamine, 10 µg/ml glycine, 15 µg/ml hypoxanthine, 5 µg/ml thymidine and 5% fetal bovine serum (Gibco BRL Life Technologies, Gaithersburg, MD). CHO cells grown in one 100 mm diameter plate (about 80-85% confluent) were transfected with linearized plasmid (15 µg). Transfections for expression of GFP alone, NT3 with GFP (NT3 described in Rosenthal *et al.,* Neuron 4: 767-773 (1990)) or DNase with GFP (DNasc described in Shak *et al*., Proc. Natl. Acad. Sci USA 87: 9188-9192 (1990)) were carried out with lipofectamine (Gibco BRL) and transfections for expression of VEGF alone (Leung *et al*., Science 246: 1306-1309 (1989)) or VEGF with GFP were carried out with SuperFect (Qiagen Inc., Santa Clarita, CA) according to manufacturers' instructions. Transfected CHO cells were grown in GHT free (medium lacking glycine, hypoxanthine and thymidine) F12/DMEM medium supplemented with 2 mM L-glutamine and 5% dialyzed fetal bovine serum.

To grow cells in methotrexate (MTX), transfected cells were put in medium containing 10 nM MTX (Sigma, St Louis, MO) and the MTX concentration was increased gradually over a period of time. For correlation studies of GFP fluorescence and productivity of the desired protein, cells were seeded at 1.5 million cells per 100 mm dish and cultured for 2 days for productivity measurements. Supernatants were harvested and the amount of the desired protein produced was measured by ELISA. Productivity (pg/cell/day) was calculated as pg/ ((Ct-C0 t/ln (Ct/C0)) where C0 and Ct were the initial and final number of cells and t was incubation time. For productivity studies of cells grown in MTX, MTX was included in the medium.

### 1.3. FACS

Flow cytometric analysis and sorting were performed as described previously using an EPICS Elite-ESP cytometer (Coulter Corp., Hialeach, FL) equipped with an argon ion laser (Ropp *et al.,* Cytometry 21: 309-317 (1995)). The excitation wavelength was 488 nm and the emission wavelength was 525 ± 25 nm. Cells in 100 mm dish were trypsinized and resuspended in 2% diafiltered FBS in PBS. Propidium iodide was added and cells were sorted at 1000-3000 cell/sec in phosphate buffered saline and collected in growth medium. Single cell cloning into 96-well plates was done using the Autoclone system equipped on the cytometer. Fluorescence intensity of clones were measured using either FACS can or FACSCalibur flow cytometer (Becton Dickinson, San Jose, CA). Calibration particles (4700 - 3.3 x 10⁵ fluorescein equivalence; Spherotech, Inc., Libertyville, IL) were used to generate a standard curve. The fluorescein equivalence of the geometric mean fluorescence intensity of cells was calculated and used in data analysis.

### 1.4. ELISA

For GFP ELISA, ELISA plates were coated with 2 µg/ml rabbit polyclonal antibody to wild type GFP (Clonetech, Palo Alto, CA) in 50 mM carbonate buffer, pH 9.6, at 4°C overnight. Plates were blocked with 0.5% bovine serum albumin in phosphate buffered saline at room temperature for 1 h. Serially diluted samples and standards (wild type GFP) in phosphate buffered saline containing 0.5% bovine serum albumin, 0.05% polysorbate 20, were added to plates and plates were incubated for 1 h. GFP bound on the plate was detected by adding biotinylated rabbit polyclonal antibody to wild type GFP followed by streptavidin peroxidase (Sigma) and 3,3',5,5'-tetramethyl benzidine (Kirkegaard & Perry Laboratories) as the substrate. Plates were washed between steps. Absorbance was read at 450 nm on a Vmax plate reader (Molecular Devices, Sunnyvale, CA). The standard curve was fitted using a four-parameter nonlinear regression curve-fitting program (developed at Genentech). Data points which fell in the linear range of the standard curve were used for calculating the GFP concentration in samples. The assay range was 0.16-10 ng/ml. NT3, DNasc or VEGF in supernatants were also measured using a sandwich type ELISA. NT3 ELISA used genuine pig polyclonal antibody to recombinant human NT3 (Genentech) for coat and biotinylated genuine pig polyclonal antibody for detection. The assay range was 0.10-6.25 ng/ml. DNase ELISA used goat polyclonal antibody to recombinant human DNase (Genentech) for coat and biotinylated rabbit polyclonal antibody for detection. The assay range was 0.39-25 ng/ml. VEGf ELISA used a monoclonal antibody to VEGF for coat and a biotinylated monoclonal antibody for detection. The assay range was 0.015-1 ng/ml (Shifren *et al*., J. Clin. Endocrinol. Metab. 81:3112-3118 (1996)).

### 1.5. RNA quantitation

Total RNA was prepared using the RNcasy mini kit (Qiagen) and the concentration was determined by absorbance. RT-PCR was carried out in a 7700 Sequence Detector (PE Applied BioSystems, Foster City, CA) using reagents purchased from PE Applied BioSystems. Sequences of the 5' and 3' end primers and probe were GTGGAGAGGGTGAAGGTGATGC (SEQ ID NO:3), CGAAAGGGCAGATTGTGTGGAC (SEQ ID NO:4), and FAM-TAACCGCTACCGGGACAGGAAAATGGT-TAMRA (SEQ ID NO:5) for GFP, respectively, AGAGTCACCGAGGGGAGTA (SEQ ID NO:6), CGTAGGTTTGGGATGTTTTG (SEQ ID NO:7) and FAM-ACGGGCAACTCTCCTGTCAAACAAT-TAMRA (SEQ ID NO:8) for NT3, respectively, AGCCACTGGGACGGAACA (SEQ ID NO:9), ACCGGGAGAAGAACCTGACA (SEQ ID NO:10), and FAM-CTGACCAGGTGTCTGCGGTGGACAG-TAMRA (SEQ ID NO:11) for DNase, respectively, and TCGCCTTGCTGCTCTACCTC (SEQ ID NO:12), GGCACACAGGATGGCTTGA (SEQ ID NO:13), and FAM-CCAAGTGGTCCCAGGCTGCACCCAT-TAMRA (SEQ ID NO:14) for VEGF, respectively. The reaction mixture had 1xBuffer A, 4 mM magnesium chloride, an optimal concentration of primers (20 nM for GFP, 50 nM for NT3 and VEGF, 25 nM for DNase), 100 nM probe, 50 ng total RNA, 0.3 mM dNTP (or 0.6 mM dUTP instead of 0.3 mM dTTP), RNase inhibitor (400 U/ml), MuLV Reverse Transcriptase (250 U/ml), TaqGold (25 U/ml) in a 50 µl reaction volume. The PCR cycle condition was 48°C, 30 min; 95°C, 10 min; 40 cycles of 95°C for 30 see and 60°C for 2 min. The amplified PCR products had the expected respective molecular weight (536 bp for GFP, 243 bp for NT3, 159 bp for DNase and 202 bp for VEGF) when analyzed on a 1% SeaKem LE, 3% NuSieve 1:3 (FMC BioProducts, Rockland, ME) agarose gel.

### 1.6. Statistical analysis

Data for correlation studies were analyzed using correlation coefficient with p-value from Fisher's r to z transformation (StatView program, Abacus Concepts, Berkeley, CA).

### 2. Results

### 2.1. Expression of GFP alone

DHFR CHO cells were transfected with the GFP expression vector. Transfected cells were grown in the GHT free medium and sorted into different fluorescence populations by FACS. To obtain high fluorescence clones, the brightest 5% of cells were sorted. Cells with six-fold higher fluorescence were obtained. After two weeks of growth, these cells were subjected to a second sort, collecting the brightest 1% of cells. After an additional two weeks of growth, the brightest 0.4% of cells were cloned in a third sort. Eighteen clones with different fluorescence intensities were selected by fluorescence microscopy. The highest fluorescence clone had a fluorescence ointensity of 1.4 mfe.

For determination of GFP concentration in these clones, lysates were prepared by incubating cells in one confluent 100 mm dish with 0.35 ml of 150 mM NaCl, 50 mM HEPES, 0.5% Triton X100 containing 1 mM AEBSF, 11 U/ml aprotinin and 50 mM leupeptin (ICN Biomedicals, Aurora, OH) on ice for 15 min. Nuclei were pelleted at 14,000 rpm in the Eppendorf centrifuge and supernatants were collected and stored frozen until assayed. GFP concentration in cell lysate was normalized by the total protein concentration measured using the BCA protein assay kit (Pierce, Rockford, IL).

Analysis of these clones demonstrated that GFP fluorescence measured by FACS correlated very well with GFP in the cellular lysate as measured by ELISA (correlation coefficient = 0.99, p < 0.0001; Fig. 7). Therefore, GFP fluorescence of the cell quantitatively represented the amount of cellular GFP protein in these clones. This is in agreement with previous reports which demonstrated that GFP fluorescence was a good measurement of total GFP content in transiently transfected CHO cells (Subramanian *et al*., J. Biotechnol 49: 137-151 (1996) and Natarajan *et al*., J. Biotechnol. 62: 29-45 (1998)). No obvious effect of GFP on CHO cell growth was observed, similar to what was reported previously (Gubin *et al*., Biochem. Biophys. Res. Commun. 236: 347-350 (1997). The FACS profiles of these clones remained the same during the two weeks studied and did not change when they were frozen and recultured.

Lysates of some selected clones were analyzed on a 16% SDS polyacrylamide gel under reducing conditions (Laemmli *et al.,* Nature 227: 680-685 (1970)). Protein blotting and probing with antibody to wild type GFP gave a single band with the expected 27 kDa molecular weight (Prasher *et al.,* Gene 111: 229-233 (1992).

Some of the high fluorescence cells obtained from the first sort were grown in increasing concentrations of MTX over two months. Clones were picked from cells grown in 50 nM (63 clones) and 100 nM (14 clones) MTX by hand and screened by fluorescence microscopy. Fluorescence intensities of six selected 50 nM clones and five selected 100 nM clones were measured by FACS. The highest fluorescence clones from 50 and 100 nM MTX had fluorescence intensities of 1.6 and 3.2 million fluorescein equivalence (mfe), respectively. In comparison, the highest fluorescence clones obtained by repeated FACS sorting had a fluorescence intensity of 1.4 mfc (Fig. 7). FACS sorting therefore selected clones with fluorescence comparable to that of clones in 50 nM MTX. The clone with 3.2 mfe fluorescence from 100 nM Mtx had 2.3 fold higher fluorescence measured by FACS and 2.2 fold more cellular GFP measured by ELISA than the clone with 1.4 mfe obtained by FACS sorting. This shows that the correlation between GFP fluorescence measured by FACS and cellular protein measured by ELISA seen in the clones obtained by FACS sorting could be extended to clones with as high as 3.2 mfe fluorescence. In addition to being less tedious, FACS sorting also avoids the heterogeneity and instability problems sometimes associated with clones selected in Mtx alone (Kaufman and Sharp, 1982; Schimke, 1992, *supra*)

### 2.2. Expression of NT3 or DNase with GFP

CHO cells were transfected with a DHFR intron vector containing cDNA encoding neuronotrophin-3 (NT3) (Rosenthal *et al.,* Neuron 4: 767-773 1990) or deoxyribonuclease (DNase) (Shak *et al.,* Proc. Natl. Acad. Sci. USA 87: 9188-9192 1990), and cDNA encoding GFP. DHFR and NT3 or DNase were expressed in one transcription unit and GFP was expressed in a second transcription unit (Fig. 1, row 4 and Fig. 6). About 2 weeks after selection or when sufficient cells we available for sorting, transfected cells were sorted and cloned by FACS. Clones with high fluorescence were obtained by sorting the brightest 5% cells at the first sort, growing the cells for two weeks, and cloning the top 4% (NT3) or 2% (DNase) cells at the second sort. Seventeen NT3-GFP clones and 15 Dnase-GFP clones with different fluorescence intensities were selected by fluorescence microscopy.

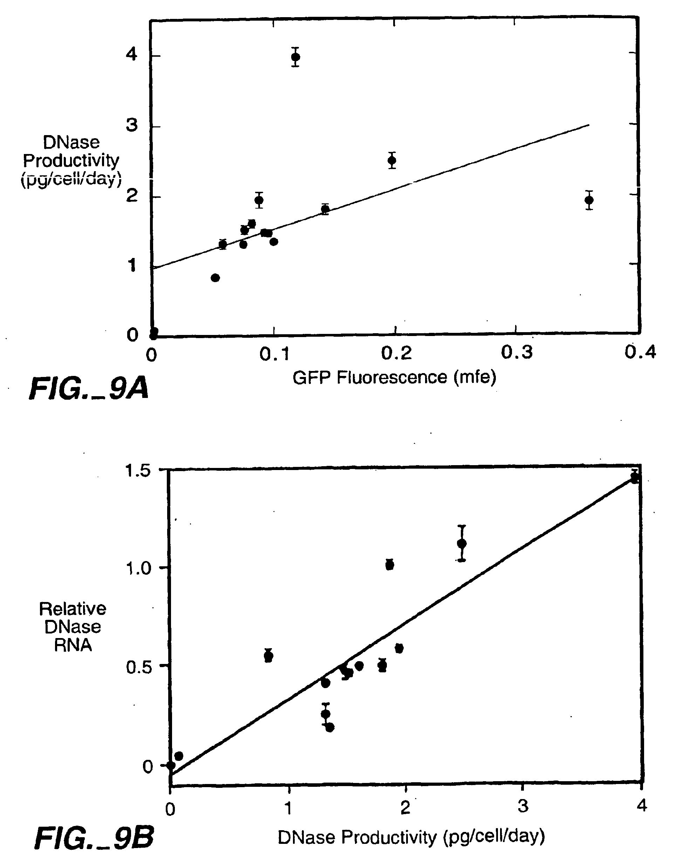

A correlation between productivity and GFP fluorescence was shown in 17 NT3-GFP producing clones (correlation coefficient = 0.68, p = 0.0018; Fig. 8A) and in 15 DNasc-GFP producing clones (correlation coefficient = 0.52, p = 0.048; Fig. 9A). (The productivity of the clone with none detectable NT3 or DNase production was calculated using the respective ELISA assay limit). Therefore, sorting cells according to GFP fluorescence by FACS increased the chance of obtaining high producing clones. NT3-GFP clones had a much lower productivity compared to DNasc-GFP clones with similar GFP fluorescence even when the molecular weight of NT3 (15 kD for a monomer; Rosenthal *et al*., Neuron 4: 767-773 1990) and DNase (29 kD; Shak *et al*., 1990) were taken into account. NT3 is known to be synthesized as a pro-protein and then processed to the mature form and has been found to be difficult to express. FACS sorting would be particularly useful to obtain high producing clones for molecules which are difficult to express.

NT3 or DNase RNA measured by RT-PCR using real-time PCR correlated with productivity very well in individual clones (correlation coefficient = 0.91, p<0.0001 for both NT3 and DNase, Figs. 8B and 9B). The amount of RNA was normalized to the amount of RNA of the clone with the highest fluorescence.

### 2.3. Comparison of obtaining high VEGF producing clones by FACS sorting vs. randomly picking clones

Vascular endothelial growth factor (VEGF) (Leung *et al*., 1989) was expressed with GFP. Transfected cells were sorted and cloned by FACS. VEGF is a potent mitogen for vascular endothelial cells *in vitro* and an angiogenic factor *in vivo.* Transfected cells were sorted and cloned by FACS. To obtain high fluorescence clones, the top 2.5% of cells were sorted and 35,000 cells were collected. After an additional two weeks of growth, the top 1.5% of cells were sorted in a second sort, collecting 50,000 cells. After an additional two weeks of growth, the top 0.5% cells were sorted in a third sort. Repeated sorting enriched the high fluorescence cell population.

The fluorescence intensity was 0.025 mfe for the high fluorescence population of the non-sorted cells (Fig. 10A), 0.12 mfe for cells from the first sort, and 1.2 mfe for cells from the second sort (Fig. 10B). The fluorescence of the clone with the highest fluorescence obtained from the third sort was 5.0 mfe (Fig. 10C). When viewed by fluorescence microscopy, very bright fluorescence could be seen distributed throughout the cytoplasm and nucleus, consistent with previous reports (Ogawa *et al*., Proc. Natl. Acad. Sci. USA 92: 11899-11903 1995; Subramanian *et al*., J. Biotechnol 49: 137-151 1996). Forty-eight clones with different fluorescence, including 15 high fluorescence clones obtained as described above, were selected by fluorescence microscopy for correlation studies.

Analysis of these cloned demonstrated that high fluorescence clones produced high amounts of VEGF and VEGF productivity correlated well with GFP fluorescence (correlation coefficient = 0.70, p< 0.0001; Fig. 11A). FACS sorting was therefore very useful for obtaining high producing clones. Additionally, VEGF productivity correlated with VEGF RNA very well (correlation coefficient = 0.90, p< 0.0001; Fig. 11B) and GFP fluorescence correlated well with GFP RNA (correlation coefficient = 0.78, p< 0.0001; Fig. 11C). In addition, VEGF RNA correlated well with GFP RNA (correlation coefficient = 0.71, p< 0.0001; Fig. 11D).

It took two months to obtain high VEGF producing clones by FACS. The FACS sorting steps might be shortened by waiting lesser time between sorts unless the two week period between sorts increased the frequency of spontaneously amplified clones (Johnson *et al*., Proc. Natl. Acad. Sci. USA 80: 3711-3715 1983).

Four VEGF-GFP clones were amplified with MTX and cloned in 500 nM MTX over two and half months. Productivity remained the same for the two clones producing 3.3 pg/cell/day, suggesting that high producing clones might require a higher concentration of MTX for amplification. Productivity decreased in some clones from the clone producing 1.9 pg/cell/day but increased to 4-5 pg/cell/day for the clone producing 1.3 pg/cell/day. Therefore, clones obtained by FACS sorting could be amplified with MTX to obtain higher producing clones.

To obtain high producing clones by the traditional way, CHO cells in 100 mm plates were transfected with the VEGF expression vector and half of he cells were plated out in six 100mm plates in GHT-free medium. Two weeks after transfection, 144 clones (24 clones from each plate) were picked randomly by hand and transferred to 96 well plates and screened for VEGF production by ELISA. Twenty-four VEGF clones were transferred to 12 well plates for further evaluation. Nine clones were selected and their productivities were measured. The highest producing clone obtained by randomly picking clones produced 0.71 pg/cell/day. In contrast, the highest producing clone obtained by FACS produced 4.4 pg/cell/day. Therefore, FACS sorting selected out high producing clones efficiently and higher producing clone was therefore obtained by FACS sorting.

To evaluate whether GFP fluorescence would be useful for selecting high producing clones in Mtx, VEGF and VEGF-GFP producing cells were grown in increasing concentrations of MTX over one and a half months. Cells were picked from seven VEGF-GFP clones (4 from 25 nM and 3 from 50 nM Mtx) selected by fluorescence microscopy. All seven produced a good amount of VEGF (0.6-3.2 pg/cell/day). In comparison, cells picked from forty-five randomly selected VEGF clones in Mtx (10 from 25 nM and 15 from 50 nM and 20 from 100 nM) produced no more than 2.4 pg/cell/day. Fluorescence microscopy therefore selected good producing cells in Mtx, indicating that FACS would be useful for further screening of cells selected in Mtx. Productivity of the top five producing clones obtained by either randomly picking clones or by FACS sorting and the top five producing populations in MTX obtained by either randomly picking populations or by fluorescence microscopy are shown in Figure 12.

### Example 2

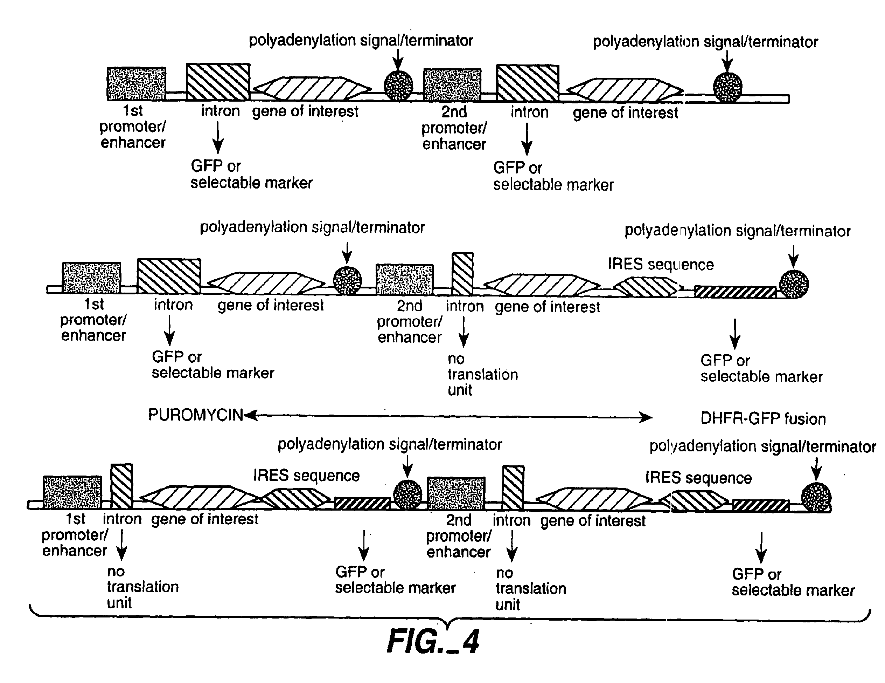

Example 2 describes the expression of an anti-IgE humanized antibody (E26) from a vector in which the antibody heavy (H) chain gene is cloned into one transcription unit and the light (L) chain gene is transcribed from a second transcription unit. For a description of the E26 antibody, see WO 99/01556 published 14 January 1999. Figure 4 shows the different configurations of the vectors used in expressing E26 antibody in DHFR DP12 CHO cells. No translation unit means that no gene insert was cloned into the intron (empty intron). As is evident from the figure, the H chain and L chain of the antibody are interchangeable in position in the two transcription units. Likewise, the positioning of the GFP and the amplifiable selectable marker in the first or second intron is also interchangeable. In one construct, the selectable marker, puromycin, was cloned within the first intron, the second intron was left empty of gene insert and a DHFR-GFP fusion gene was inserted 3' of the IRES (Fig. 4, middle row).

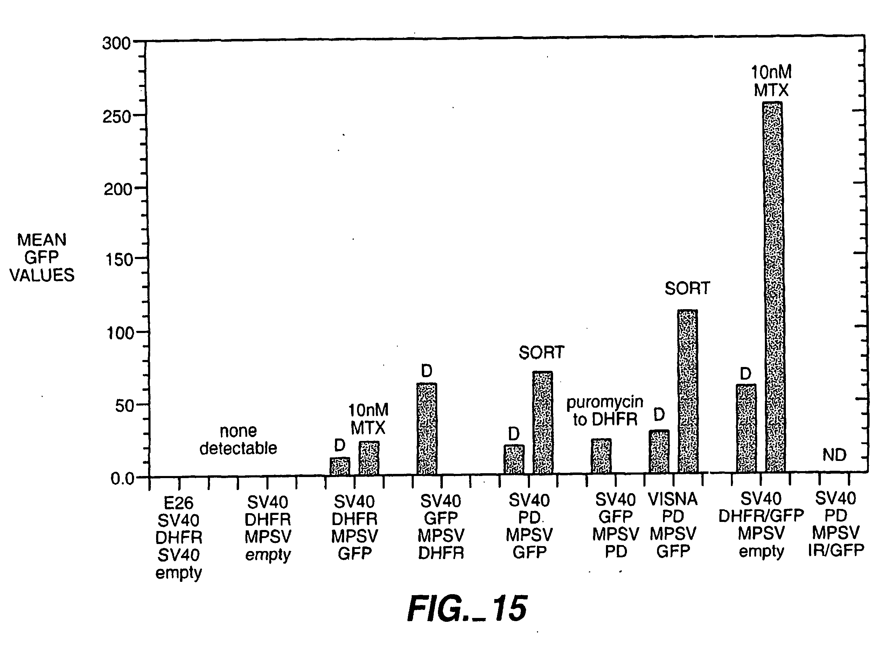

Figure 15 shows the results of GFP FACS analysis of E26 antibody expressing cell pools. The mean GFP values (log-GFP) was determined across 100% gated cells. Antibody expression levels were also assayed under identical conditions for each pool after 48 hours (Figure 14) and compared for correlation to GFP expression. Pools selected in 10nM mtx (10nM) for greater stringency versus those selected in GHT minus media, a minimal stringency standard for the DHFR protocol (D), showed increases in both productivity and mean GFP fluorescence. Two of the GHT minus-selected pools were also sorted and cells from the top 5% fluorescence values were expanded and reevaluated for antibody expression and GFP fluorescence. In each case, antibody expression improved with fluorescence (sort). In all cases, the placement of the selectable marker (DHFR or puromycin-DHFR fusion) in the intron 5' to the H chain and the GFP gene in the intron 5' of the L chain showed consistently correlative relationships in expression and GFP determination.

## Claims

1. A polynucleotide comprising:
a) an amplifiable selectable gene;
b) a green fluorescent protein (GFP) gene; and
c) a selected sequence encoding a desired product, the selected sequence operably linked to either the amplifiable selectable gene or to the GFP gene, and to a promoter, wherein the polynucleotide comprises
a first transcription unit comprising a first promoter followed by an intron and the selected sequence; and
a second transcription unit comprising a second promoter and
an intron 3' of the second promoter;
wherein the intron in the first transcription unit is the first intron, and the intron in the second transcription unit is the second intron, and wherein each of the first and the second introns is defined by a 5' splice donor site and a 3' splice acceptor site providing a splicing efficiency of at least 95% as determined by quantitative PCR; and wherein either
(i) the amplifiable selectable gene is positioned in the first intron and operably linked to the first promoter, and the GFP gene is positioned in or 3' of the second intron and operably linked to the second promoter; or
(ii) the GFP gene is positioned in the first intron and operably linked to the first promoter and the amplifiable selectable gene is positioned in the second transcription unit and operably linked to the second promoter.

2. The polynucleotide of claim 1, wherein the amplifiable selectable gene is selected from the group of consisting of the genes encoding dihydrofolate reductase (DHFR) and glutamine synthetase.

3. The polynucleotide of claim 1 or claim 2, wherein the GFP gene encodes a mutant GFP exhibiting a higher fluorescence intensity than the wild-type GFP.

4. The polynucleotide of claim 3, wherein the mutant GFP is GFP-S65T having a serine to threonine substitution in amino acid 65 of the wild-type GFP of *Aequorea victoria.*

5. The polynucleotide of any of claims 1 to 4, wherein the amplifiable selectable gene is positioned in the intron in the first transcription unit wherein the amplifiable selectable gene and the selected sequence are both operably linked to the first promoter; and the GFP gene is positioned 3' of the second intron and operably linked to the second promoter in the second transcription unit.

6. The polynucleotide of any of claims 1 to 4, wherein the amplifiable selectable gene is positioned in the first intron and operably linked to the first promoter, and the GFP gene is positioned in the second intron and operably linked to the second promoter.

7. The polynucleotide of any of claims 1 to 4, wherein the GFP gene is positioned in the first intron and operably linked to the first promoter, and the amplifiable selectable gene is positioned in the second intron and operably linked to the second promoter.

8. The polynucleotide of any of claims 1 to 7, wherein the second transcription unit further comprises a selected sequence 3' of the second intron, wherein the selected sequence in the first transcription unit is the first selected sequence, and the selected sequence in the second transcription unit is the second selected sequence wherein the second selected sequence is operably linked to the second promoter and encodes a second desired product.

9. The polynucleotide of claim 8, further comprising an IRES 3' of the second selected sequence.

10. The polynucleotide of claim 9, wherein the amplifiable selectable gene is positioned in the first intron and operably linked to the first promoter, and the GFP gene is positioned 3' of the IRES and operably linked to the second promoter.

11. The polynucleotide of claim 8, wherein the first transcription unit further comprises an IRES 3' of the first selected sequence.

12. The polynucleotide of claim 11, wherein the second transcription unit further comprises an IRES 3' of the second selected sequence, wherein the IRES in the first transcription unit is the first IRES, and the IRES in the second transcription unit is the second IRES.

13. The polynucleotide of any of the preceding claims, wherein the first promoter and the second promoter are the CMV or the SV40 promoter.

14. The polynucleotide of any of the preceding claims, wherein at least one of the promoters is inducible.

15. The polynucleotide of claim 13, wherein the CMV promoter is the human cytomegalovirus immediate early (CMV) promoter.

16. A host cell comprising the polynucleotide of any of the preceding claims.

17. The host cell of claim 16, wherein the cell is a mammalian cell.

18. The host cell of claim 17 wherein the mammalian cell is a Chinese Hamster Ovary (CHO) cell.

19. The host cell of claim 18, wherein the amplifiable selectable gene is the DHFR gene and the CHO cell has a DHFR⁻ phenotype.

20. A method of producing a desired protein comprising introducing the polynucleotide of any one of claims 1 to 15 into a suitable eukaryotic host cell, culturing the eukaryotic host cell under conditions so as to express the desired protein, and recovering the desired protein.

## Patentansprüche

1. Polynucleotid, umfassend:
a) ein amplifizierbares selektierbares Gen;
b) ein grün fluoreszierendes Protein- (GFP) Gen; und
c) eine selektierte Sequenz, die für ein erwünschtes Produkt kodiert, wobei die selektierte Sequenz operabel entweder an das amplifizierbare selektierbare Gen oder das GFP-Gen und an einen Promotor gebunden ist, worin das Polynucleotid
eine erste Transkriptionseinheit, die einen ersten Promotor gefolgt von einem Intron und der selektierten Sequenz; und
eine zweite Transkriptionseinheit, die einen zweiten Promotor und ein Intron 3' des zweiten Promotors umfasst;
worin das Intron in der ersten Transkriptionseinheit das erste Intron ist und das Intron in der zweiten Transkriptionseinheit das zweite Intron ist und worin beide von erstem und zweitem Intron durch eine 5'-Spleißdonorstelle und eine 3'-Spleißakzeptorstelle definiert sind, wodurch eine Spleißwirksamkeit von zumindest 95 %, bestimmt durch quantitative PCR, bereitgestellt wird; und worin entweder
(i) das amplifizierbare selektierbare Gen im ersten Intron angeordnet und an den ersten Promotor operabel gebunden ist und das GFP-Gen im oder 3' vom zweiten Intron angeordnet und an den zweiten Promotor operabel gebunden ist; oder
(ii) das GFP-Gen im ersten Intron angeordnet und an den ersten Promotor operabel gebunden ist und das amplifizierbare selektierbare Gen in der zweiten Transkriptionseinheit angeordnet und an den zweiten Promotor operabel gebunden ist.

2. Polynucleotid nach Anspruch 1, worin das amplifizierbare selektierbare Gen aus der aus den für Dihydrofolatreductase (DHFR) und Glutaminsynthetase kodierenden Genen bestehenden Gruppe ausgewählt ist.

3. Polynucleotid nach Anspruch 1 oder Anspruch 2, worin das GFP-Gen für ein mutiertes GFP kodiert, das eine höhere Fluoreszenzaktivität als das Wildtyp-GFP aufweist.

4. Polynucleotid nach Anspruch 3, worin das mutierte GFP GFP-S65T mit einer Serin-zu-Threonin-Substitution in Aminosäure 65 des Wildtyp-GFP von Aequorea victoria ist.

5. Polynucleotid nach einem der Ansprüche 1 bis 4, worin das amplifizierbare selektierbare Gen im Intron der ersten Transkriptionseinheit angeordnet ist, worin das amplifizierbare selektierbare Gen und die selektierte Sequenz beide operabel an den ersten Promotor gebunden sind; und das GFP-Gen 3' vom zweiten Intron angeordnet und operabel an den zweiten Promotor in der zweiten Transkriptionseinheit gebunden ist.

6. Polynucleotid nach einem der Ansprüche 1 bis 4, worin das amplifizierbare selektierbare Gen im ersten Intron angeordnet und operabel an den ersten Promotor gebunden ist und das GFP-Gen im zweiten Intron angeordnet und operabel an den zweiten Promotor gebunden ist.

7. Polynucleotid nach einem der Ansprüche 1 bis 4, worin das GFP-Gen im ersten Intron angeordnet und operabel an den ersten Promotor gebunden ist und das amplifizierbare selektierbare Gen im zweiten Intron angeordnet und operabel an den zweiten Promotor gebunden ist.

8. Polynucleotid nach einem der Ansprüche 1 bis 7, worin die zweite Transkriptionseinheit weiters eine selektierte Sequenz 3' vom zweiten intron umfasst, worin die selektierte Sequenz in der ersten Transkriptionseinheit die erste selektierte Sequenz ist und die selektierte Sequenz in der zweiten Transkriptionseinheit die zweite selektierte Sequenz ist, worin die zweite selektierte Sequenz operabel an den zweiten Promotor gebunden ist und für ein zweites erwünschtes Produkt kodiert.

9. Polynucleotid nach Anspruch 8, weiters umfassend ein IRES 3' von der zweiten selektierten Sequenz.

10. Polynucleotid nach Anspruch 9, worin das amplifizierbare selektierbare Gen im ersten Intron angeordnet und operabel an den ersten Promotor gebunden ist und das GFP-Gen 3' vom IRES angeordnet und operabel an den zweiten Promotor gebunden ist.

11. Polynucleotid nach Anspruch 8, worin die erste Transkriptionseinheit weiters ein IRES 3' von der ersten selektierten Sequenz umfasst.

12. Polynucleotid nach Anspruch 11, worin die zweite Transkriptionseinheit weiters ein IRES 3' von der zweiten selektierten Sequenz umfasst, worin das IRES in der ersten Transkriptionseinheit das erste IRES und das IRES in der zweiten Transkriptionseinheit das zweite IRES ist.

13. Polynucleotid nach einem der vorangehenden Ansprüche, worin der erste Promotor und der zweite Promotor der CMV- oder der SV40-Promotor sind.

14. Polynucleotid nach einem der vorangehenden Ansprüche, worin zumindest einer der Promotoren induzierbar ist.

15. Polynucleotid nach Anspruch 13, worin der CMV-Promotor der menschliche, unmittelbar frühe Zytomegalievirus- (CMV-) Promotor ist.

16. Wirtszelle, umfassend das Polynucleotid nach einem der vorangehenden Ansprüche.

17. Wirtszelle nach Anspruch 16, worin die Zelle eine Säugetierzelle ist.

18. Wirtszelle nach Anspruch 17, worin die Säugetierzelle eine Chinahamster-Eierstock- (CHO-) Zelle ist.

19. Wirtszelle nach Anspruch 18, worin das amplifizierbare selektierbare Gen das DHFR-Gen ist und die CHO-Zelle einen DHFR⁻-Phänotyp aufweist.

20. Verfahren zur Herstellung eines erwünschten Proteins, umfassend das Einführen des Polynucleotids nach einem der Ansprüche 1 bis 15 in eine geeignete eukaryotische Wirtszelle, das Kultivieren der eukaryotischen Wirtszelle unter Bedingungen, um das erwünschte Protein zu exprimieren, und das Gewinnen des erwünschten Proteins.

## Revendications

1. Polynucléotide comprenant :
a) un gène amplifiable sélectionnable ;
b) un gène de protéine fluorescente verte (GFP) ; et
c) une séquence choisie codant pour un produit souhaité, la séquence choisie étant opérationnellement liée soit au gène amplifiable sélectionnable, soit au gène de la GFP, et à un promoteur, le polynucléotide comprenant
une première unité de transcription comprenant un premier promoteur suivi d'un intron et de la séquence choisie ; et
une deuxième unité de transcription comprenant un deuxième promoteur et un intron en 3' du deuxième promoteur ;
dans lequel l'intron dans la première unité de transcription représente le premier intron, et l'intron dans la deuxième unité de transcription représente le deuxième intron, et dans lequel chacun des premier et deuxième introns est défini par un site en 5' donneur d'épissage et un site en 3' accepteur d'épissage fournissant une efficacité d'épissage d'au moins 95 %, comme déterminé par PCR quantitative ; et dans lequel soit
(i) le gène amplifiable sélectionnable est situé dans le premier intron et opérationnellement lié au premier promoteur, et le gène de la GFP est situé dans ou en 3' du deuxième intron et opérationnellement lié au deuxième promoteur ; soit
(ii) le gène de la GFP est situé dans le premier intron et opérationnellement lié au premier promoteur, et le gène amplifiable sélectionnable est situé dans la deuxième unité de transcription et opérationnellement lié au deuxième promoteur.

2. Polynucléotide selon la revendication 1, dans lequel le gène amplifiable sélectionnable est choisi dans le groupe constitué par les gènes codant pour la dihydrofolate-réductase (DHFR) et la glutamine-synthétase.

3. Polynucléotide selon la revendication 1 ou la revendication 2, dans lequel le gène de la GFP code pour une GFP mutante manifestant une intensité de fluorescence supérieure à celle de la GFP de type sauvage.

4. Polynucléotide selon la revendication 3, dans lequel la GFP mutante est la GFP -S65T ayant une substitution de la sérine en thréonine dans l'aminoacide 65 de la GFP de type sauvage de *Aequorea victoria.*

5. Polynucléotide selon l'une quelconque des revendications 1 à 4, dans lequel le gène amplifiable sélectionnable est situé dans l'intron dans la première unité de transcription, le gène amplifiable sélectionnable et la séquence choisie étant l'un et l'autre opérationnellement liés au premier promoteur ; et le gène de la GFP est situé en 3' du deuxième intron et opérationnellement lié au deuxième promoteur dans la deuxième unité de transcription.

6. Polynucléotide selon l'une quelconque des revendications 1 à 4, dans lequel le gène amplifiable sélectionnable est situé dans le premier intron et opérationnellement lié au premier promoteur, et le gène de la GFP est situé dans le deuxième intron et opérationnellement lié au deuxième promoteur.

7. Polynucléotide selon l'une quelconque des revendications 1 à 4, dans lequel le gène de la GFP est situé dans le premier intron et opérationnellement lié au premier promoteur, et le gène amplifiable sélectionnable est situé dans le deuxième intron et opérationnellement lié au deuxième promoteur.

8. Polynucléotide selon l'une quelconque des revendications 1 à 7, dans lequel la deuxième unité de transcription comprend en outre une séquence choisie en 3' du deuxième intron, dans lequel la séquence choisie dans la première unité de transcription représente la première séquence choisie, et la séquence choisie dans la deuxième unité de transcription représente la deuxième séquence choisie, la deuxième séquence choisie étant opérationnellement liée au deuxième promoteur et codant pour un deuxième produit souhaité.

9. Polynucléotide selon la revendication 8, comprenant en outre une IRES en 3' de la deuxième séquence choisie.

10. Polynucléotide selon la revendication 9, dans lequel le gène amplifiable sélectionnable est situé dans le premier intron et opérationnellement lié au premier promoteur, et le gène de la GFP est situé en 3' de l'IRES et opérationnellement lié au deuxième promoteur.

11. Polynucléotide selon la revendication 8, dans lequel la première unité de transcription comprend en outre une IRES en 3' de la première séquence choisie.

12. Polynucléotide selon la revendication 11, dans lequel la deuxième unité de transcription comprend en outre une IRES en 3' de la deuxième séquence choisie, dans lequel l'IRES dans la première unité de transcription représente la première IRES, et l'IRES dans la deuxième unité de transcription représente la deuxième IRES.

13. Polynucléotide selon l'une quelconque des revendications précédentes, dans lequel le premier promoteur et le deuxième promoteur sont le promoteur du CMV ou du SV40.

14. Polynucléotide selon l'une quelconque des revendications précédentes, dans lequel au moins l'un des promoteurs est inductible.

15. Polynucléotide selon la revendication 13, dans lequel le promoteur du CMV est le promoteur (CMV) précoce immédiat du cytomégalovirus humain.

16. Cellule hôte comprenant le polynucléotide selon l'une quelconque des revendications précédentes.

17. Cellule hôte selon la revendication 16, ladite cellule étant une cellule de mammifère.

18. Cellule hôte selon la revendication 17, ladite cellule de mammifère étant une cellule (CHO) d'ovaire de hamster chinois.

19. Cellule hôte selon la revendication 18, dans laquelle le gène amplifiable sélectionnable est le gène de la DHFR et la cellule CHO a un phénotype DHFR⁻.

20. Procédé de production d'une protéine souhaitée comprenant l'introduction du polynucléotide selon l'une quelconque des revendications 1 à 15 dans une cellule hôte eucaryote appropriée, la culture de la cellule hôte eucaryote dans des conditions adaptées pour exprimer la protéine souhaitée, et la récupération de la protéine souhaitée.


### Sequence Listing

<110> Genentech, Inc. et al.
<120> EXPRESSION VECTORS AND METHODS
<130> P1746R1 PCT
<141> 2000-07-11
<150> US 60/143,360
   <151> 1999-07-12
<160> 14
<210> 1
   <211> 218
   <212> PRT
   <213> Artificial sequence
<220>
   <223> mouse-human chimera
<400> 1
   Asp Ile Gln Leu Thr Gln Ser Pro Ser Ser Leu Ser Ala Ser Val
   1               5                   10                  15
   Gly Asp Arg Val Thr Ile Thr Cys Arg Ala Ser Lys Pro Val Asp
               20                  25                  30
   Gly Glu Gly Asp Ser Tyr Leu Asn Trp Tyr Gln Gln Lys Pro Gly
           35                  40                  45
   Lys Ala Pro Lys Leu Leu Ile Tyr Ala Ala Ser Tyr Leu Glu Ser
       50                  55                  60
   Gly Val Pro Ser Arg Phe Ser Gly Ser Gly Ser Gly Thr Asp Phe
   65                  70                  75
   Thr Leu Thr Ile Ser Ser Leu Gln Pro Glu Asp Phe Ala Thr Tyr
                   80                  85                  90
   Tyr Cys Gln Gln Ser His Glu Asp Pro Tyr Thr Phe Gly Gln Gly
               95                  100                 105
   Thr Lys Val Glu Ile Lys Arg Thr Val Ala Ala Pro Ser Val Phe
           110                 115                 120
   Ile Phe Pro Pro Ser Asp Glu Gln Leu Lys Ser Gly Thr Ala Ser
       125                 130                 135
   Val Val Cys Leu Leu Asn Asn Phe Tyr Pro Arg Glu Ala Lys Val
   140                 145                 150
   Gln Trp Lys Val Asp Asn Ala Leu Gln Ser Gly Asn Ser Gln Glu
                   155                 160                 165
   Ser Val Thr Glu Gln Asp Ser Lys Asp Ser Thr Tyr Ser Leu Ser
               170                 175                 180
   Ser Thr Leu Thr Leu Ser Lys Ala Asp Tyr Glu Lys His Lys Val
           185                 190                 195
   Tyr Ala Cys Glu Val Thr His Gln Gly Leu Ser Ser Pro Val Thr
       200                 205                 210
   Lys Ser Phe Asn Arg Gly Glu Cys
   215
<210> 2
   <211> 451
   <212> PRT
   <213> Artificial sequence
<220>
   <223> mouse-human chimera
<400> 2
   Glu Val Gln Leu Val Glu Ser Gly Gly Gly Leu Val Gln Pro Gly
   1 5 10 15
   Gly Ser Leu Arg Leu Ser Cys Ala Val Ser Gly Tyr Ser Ile Thr
   20 25 30
   Ser Gly Tyr Ser Trp Asn Trp Ile Arg Gln Ala Pro Gly Lys Gly
   35 40 45
   Leu Glu Trp Val Ala Ser Ile Thr Tyr Asp Gly Ser Thr Asn Tyr
   50 55 60
   Asn Pro Ser Val Lys Gly Arg Ile Thr Ile Ser Arg Asp Asp Ser
   65 70 75
   Lys Asn Thr Phe Tyr Leu Gln Met Asn Ser Leu Arg Ala Glu Asp
   80 85 90
   Thr Ala Val Tyr Tyr Cys Ala Arg Gly Ser His Tyr Phe Gly His
   95 100 105
   Trp His Phe Ala Val Trp Gly Gln Gly Thr Leu Val Thr Val Ser
   110 115 120
   Ser Ala Ser Thr Lys Gly Pro Ser Val Phe Pro Leu Ala Pro Ser
   125 130 135
   Ser Lys Ser Thr Ser Gly Gly Thr Ala Ala Leu Gly Cys Leu Val
   140 145 150
   Lys Asp Tyr Phe Pro Glu Pro Val Thr Val Ser Trp Asn Ser Gly
   155 160 165
   Ala Leu Thr Ser Gly Val His Thr Phe Pro Ala Val Leu Gln Ser
   170 175 180
   Ser Gly Leu Tyr Ser Leu Ser Ser Val Val Thr Val Pro Ser Ser
   185 190 195
   Ser Leu Gly Thr Gln Thr Tyr Ile Cys Asn Val Asn His Lys Pro
   200 205 210
   Ser Asn Thr Lys Val Asp Lys Lys Val Glu Pro Lys Ser Cys Asp
   215 220 225
   Lys Thr His Thr Cys Pro Pro Cys Pro Ala Pro Glu Leu Leu Gly
   230 235 240
   Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val
   260 265 270
   Ser His Glu Asp Pro Glu Val Lys Phe Asn Trp Tyr Val Asp Gly
                   275                 280                 285
   Val Glu Val His Asn Ala Lys Thr Lys Pro Arg Glu Glu Gln Tyr
                   290                 295                 300
   Asn Ser Thr Tyr Arg Val Val Ser Val Leu Thr Val Leu His Gln
                   305                 310                 315
   Asp Trp Leu Asn Gly Lys Glu Tyr Lys Cys Lys Val Ser Asn Lys
                   320                 325                 330
   Ala Leu Pro Ala Pro Ile Glu Lys Thr Ile Ser Lys Ala Lys Gly
                   335                 340                 345
   Gln Pro Arg Glu Pro Gln Val Tyr Thr Leu Pro Pro Ser Arg Glu
                   350                 355                 360
   Glu Met Thr Lys Asn Gln Val Ser Leu Thr Cys Leu Val Lys Gly
                   365                 370                 375
   Phe Tyr Pro Ser Asp Ile Ala Val Glu Trp Glu Ser Asn Gly Gln
                   380                 385                 390
   Pro Glu Asn Asn Tyr Lys Thr Thr Pro Pro Val Leu Asp Ser Asp
                   395                 400                 405
   Gly Ser Phe Phe Leu Tyr Ser Lys Leu Thr Val Asp Lys Ser Arg
                   410                 415                 420
   Trp Gln Gln Gly Asn Val Phe Ser Cys Ser Val Met His Glu Ala
                   425                 430                 435
   Leu His Asn His Tyr Thr Gln Lys Ser Leu Ser Leu Ser Pro Gly
                   440                 445                 450
   Lys
<210> 3
   <211> 22
   <212> DNA
   <213> Artificial sequence
<220>
   <223> PCR primer and probe
<400> 3
   gtggagaggg tgaaggtgat gc 22
<210> 4
   <211> 22
   <212> DNA
   <213> Artificial sequence
<220>
   <223> PCR primer and probe
<400> 4
   cgaaagggca gattgtgtgg ac 22
<210> 5
   <211> 27
   <212> DNA
   <213> Artificial sequence
<220>
   <223> PCR primer and probe
<400> 5
   taaccgctac cgggacagga aaatggt 27
<210> 6
   <211> 19
   <212> DNA
   <213> Artificial sequence
<220>
   <223> PCR primer and probe
<400> 6
   agagtcaccg aggggagta 19
<210> 7
   <211> 20
   <212> DNA
   <213> Artificial sequence
<220>
   <223> PCR primer and probe
<400> 7
   cgtaggtttg ggatgttttg 20
<210> 8
   <211> 25
   <212> DNA
   <213> Artificial sequence
<220>
   <223> PCR primer and probe
<400> 8
   acgggcaact ctcctgtcaa acaat 25
<210> 9
   <211> 18
   <212> DNA
   <213> Artificial sequence
<220>
   <223> PCR primer and probe
<400> 9
   agccactggg acggaaca 18
<210> 10
   <211> 20
   <212> DNA
   <213> Artificial sequence
<220>
   <223> PCR primer and probe
<400> 10
   accgggagaa gaacctgaca 20
<210> 11
   <211> 25
   <212> DNA
   <213> Artificial sequence
<220>
   <223> PCR primer and probe
<400> 11
   ctgaccaggt gtctgcggtg gacag 25
<210> 12
   <211> 20
   <212> DNA
   <213> Artificial sequence
<220>
   <223> PCR primer and probe
<400> 12
   tcgccttgct gctctacctc 20
<210> 13
   <211> 19
   <212> DNA
   <213> Artificial sequence
<220>
   <223> PCR primer and probe
<400> 13
   ggcacacagg atggcttga 19
<210> 14
   <211> 25
   <212> DNA
   <213> Artificial sequence
<220>
   <223> PCR primer and probe
<400> 14
   ccaagtggtc ccaggctgca cccat 25